(19) Europäisches Patentamt
European Patent Office
Office européen des brevets

(11) Veröffentlichungsnummer: **0 192 111**
**B1**

(12) **EUROPÄISCHE PATENTSCHRIFT**

(45) Veröffentlichungstag der Patentschrift: **16.05.90**

(51) Int. Cl.[5]: **C07C 275/70**, A01N 47/42

(21) Anmeldenummer: **86101344.9**

(22) Anmeldetag: **03.02.86**

(54) **Isoharnstoffe, Verfahren zu ihrer Herstellung und ihre Verwendung zur Bekämpfung unerwünschten Pflanzenwuchses.**

(30) Priorität: **09.02.85 DE 3504453**

(43) Veröffentlichungstag der Anmeldung: **27.08.86 Patentblatt 86/35**

(45) Bekanntmachung des Hinweises auf die Patenterteilung: **16.05.90 Patentblatt 90/20**

(84) Benannte Vertragsstaaten: **BE CH DE FR GB IT LI NL**

(56) Entgegenhaltungen:
**AT-B- 222 131**
**DE-B- 1 138 039**
**DE-C- 1 137 000**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen(DE)**

(72) Erfinder: **Kiehs, Karl, Dr., Sudetenstrasse 22, D-6840 Lampertheim(DE)**
Erfinder: **Wuerzer, Bruno, Dr., Ruedigerstrasse 13, D-6701 Otterstadt(DE)**
Erfinder: **Meyer, Norbert, Dr., Dossenheimer Weg 22, D-6802 Ladenburg(DE)**

**EP 0 192 111 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Isoharnstoffe, deren Herstellung, Herbizide, die diese Verbindungen als Wirkstoffe enthalten, sowie deren Verwendung zur Bekämpfung unerwünschten Pflanzenwuchses.

Isoharnstoffe, die sich zur Anwendung als Herbizide eignen, sind aus der DE-PS 1 137 000 bekannt. In dieser Patentschrift werden nur wenige Verbindungen beschrieben, deren herbizide Wirkung sich auf die Vorauflaufapplikation beschränkt.

Es wurde gefunden, daß Isoharnstoffe der Formel I,

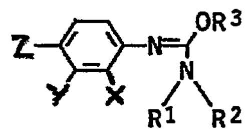

I

in der die Substituenten folgende Bedeutung haben:

$R^1$ eine $C_1$–$C_3$-Alkylgruppe,

$R^2$ eine $C_1$–$C_3$-Alkylgruppe oder eine Methoxygruppe,

$R^3$ ein Phenylrest, der ein oder zwei Halogenatome und/oder $C_1$–$C_5$-Alkylgruppen tragen kann, wobei der Phenylrest durch ein oder zwei der folgenden Gruppen substituiert ist:
$C_1$–$C_4$-Alkoxy, $C_1$–$C_3$-Halogenalkoxy, 1-($C_2$–$C_3$-Alkoxycarbonyl)-$C_1$–$C_2$-alkoxy und/oder –$NHR^4$ oder

ein Benzylrest, der bis zu drei der folgenden Gruppen tragen kann: Halogen, Hydroxy, Nitro, $C_1$–$C_5$-Alkyl, $C_1$–$C_4$-Alkoxy, $C_1$–$C_3$-Halogenalkoxy, 1-($C_2$–$C_3$-Alkoxycarbonyl)-$C_1$–$C_2$-alkoxy und/oder –$NHR^4$, wobei

$R^4$ eine $C_2$–$C_4$-Alkoxycarbonylgruppe, eine $C_2$–$C_4$-Alkylcarbonylgruppe, eine $C_4$–$C_7$-Cycloalkylcarbonylgruppe, eine N,N-Di-$C_1$–$C_4$-alkylaminocarbonylgruppe oder eine $C_2$–$C_3$-Alkoxycarbonyl-$C_1$–$C_4$-alkylgruppe, wobei diese Gruppen bis zu drei Halogenatome tragen können, bedeutet und

X, Y und Z unabhängig voneinander: Wasserstoff, Halogenatom, $C_1$–$C_4$-Alkylgruppe, $C_1$–$C_4$-Alkoxygruppe, $C_1$–$C_3$-Halogenalkylgruppe, Phenoxygruppe und/oder Phenyl-$C_1$–$C_4$-alkoxygruppe, wobei die aromatischen Gruppen bis zu drei der folgenden Substituenten tragen können: Halogen und/oder $C_1$–$C_4$-Alkyl,

herbizid, insbesondere im Nachauflaufverfahren, sehr gut wirksam und bekannten Wirkstoffen ähnlicher Struktur überlegen sind.

In Formel I bedeuten X, Y und Z Wasserstoff, Halogen, beispielsweise Chlor, Fluor, unverzweigtes oder verzweigtes $C_1$–$C_4$-Alkyl, beispielsweise Methyl, Ethyl, Isopropyl, t-Butyl, unverzweigtes oder verzweigtes $C_1$–$C_4$-Alkoxy, beispielsweise Methoxy, Ethoxy, i-Propoxy, unverzweigtes oder verzweigtes $C_1$–$C_3$-Halogenalkyl, beispielsweise Trifluormethyl, Aryl-$C_1$–$C_4$-alkoxy, wobei der Arylrest $C_1$–$C_4$-Alkylgruppen, beispielsweise Methylgruppen, tragen kann, beispielsweise 2-(4-Methylphenyl)-ethoxy, 2-(4-Ethylphenyl)-ethoxy, 2-(3,4-Dimethylphenyl)-ethoxy, 2-(4-n-Propylphenyl)-ethoxy oder gegebenenfalls durch Halogen oder $C_1$–$C_4$-Alkyl substituiertes Phenoxy, beispielsweise 4-Chlorphenoxy, 4-Fluorphenoxy, 4-Bromphenoxy, 3,4-Dimethylphenoxy, 4-i-Propylphenoxy, 4-t-Butylphenoxy,

$R^1$ unverzweigtes oder verzweigtes $C_1$–$C_3$-Alkyl, beispielsweise Methyl, Ethyl, Isopropyl, vorzugsweise Methyl,

$R^2$ unverzweigtes oder verzweigtes $C_1$–$C_3$-Alkyl, beispielsweise Methyl, Ethyl, Isopropyl, vorzugsweise Methyl oder Methoxy und

$R^3$ einen Phenylring, der einfach oder zweifach durch unverzweigte oder verzweigte Alkylreste, wie Methyl, Ethyl, i-Propyl und/oder Halogen, wie Fluor, Chlor, Brom, substituiert sein kann.
Dieser Phenylring trägt zusätzlich einen der folgenden Substituenten:

– unverzweigte oder verzweigte $C_1$–$C_4$-Alkoxyreste wie Methoxy, t-Butoxy, i-Propoxy,
– unverzweigte oder verzweigte $C_1$–$C_3$-Halogenalkoxyreste, wie Trifluormethoxy, 1,1,2,2-Tetrafluorethoxy, 1,1,2-Trifluor-2-chlorethoxy, 1,1,2-Trifluor-2-bromethoxy,
– 1-($C_2$–$C_3$-Alkoxycarbonyl)-$C_1$–$C_2$-alkoxy, wie Methoxycarbonyl-methoxy, Ethoxycarbonyl-methoxy, 1-Methoxycarbonyl-ethoxy, 1-Ethoxycarbonyl-ethoxy, oder
– einen Rest der Formel $NHR^4$;

einen Benzylrest, der bis zu dreifach durch die obengenannten Halogenatome, Alkylreste, Alkoxyreste, Halogenalkoxyreste, Alkoxycarbonyl-alkoxyreste, –$NHR^4$-Reste sowie außerdem Hydroxygruppen und/oder Nitrogruppen substituiert sein kann.

$R^4$ steht für unverzweigtes oder verzweigtes $C_2$–$C_4$-Alkoxycarbonyl-, $C_2$–$C_4$-Alkylcarbonyl-, $C_4$–$C_7$-Cycloalkylcarbonyl-, N-($C_1$–$C_4$-Alkyl)-N-($C_1$–$C_4$-alkoxy)-amino-carbonyl-, N,N-Di-($C_1$–$C_4$-alkyl)-amino-carbonyl- oder $C_2$–$C_3$-Alkoxycarbonyl-$C_1$–$C_4$-alkylreste, beispielsweise für Methoxycarbonyl,

Ethoxycarbonyl, i-Propoxycarbonyl, n-Butoxycarbonyl, Methylcarbonyl, Ethylcarbonyl, n-Propylcarbonyl, t-Butylcarbonyl, Cyclopropylcarbonyl, Cyclopentylcarbonyl, Cyclohexylcarbonyl, Di-methylaminocarbonyl, Diethylaminocarbonyl, Di-n-propylaminocarbonyl, N-Methyl-N-propylaminocarbonyl, Methoxycarbonylmethyl, 2-Methoxycarbonylethyl, 2-Ethoxycarbonylethyl, Ethoxycarbonylmethyl, 3-Methoxycarbonylpropyl, 3-Ethoxycarbonylpropyl, Propoxycarbonylmethyl, Di-n-butylaminocarbonyl, Di-i-propylaminocarbonyl, N-Methyl-N-methoxyamino-carbonyl.

Beispiele für Reste für $R^3$ sind 2-Methoxyphenyl, 3-Methoxyphenyl, 4-Methoxyphenyl, 3,4-Dimethoxyphenyl, 2-i-Propoxyphenyl, 4-i-Propoxyphenyl, 4-t-Butoxyphenyl, 2-Trifluormethylphenyl, 3-Trifluormethylphenyl, 4-Trifluormethylphenyl, 3-(N,N-Dimethylcarbamoylamino)-phenyl, 4-(N,N-Dimethylcarbamoylamino)-phenyl, 2-Chlor-4-(N,N-dimethylcarbamoylamino)-phenyl, 2-Fluor-4-(N,N-dimethylcarbamoylamino)-phenyl, 2-Methyl-4-(N,N-dimethylcarbamoylamino)-phenyl, 2,6-Dichlor-4-(N,N-dimethylcarbamoylamino)-phenyl, 2-Methyl-5-(N,N-dimethylcarbamoylamino)-phenyl, 2-Chlor-5-(N,N-dimethylcarbamoylamino)-phenyl, 2-Fluor-5-(N,N-dimethylcarbamoylamino)-phenyl, 3-(N-Methyl-N-methoxycarbamoylamino)-phenyl, 4-(N-Methyl-N-methoxycarbamoylamino)-phenyl, 2-Chlor-4-(N-methyl-N-methoxycarbamoylamino)-phenyl, 2-Fluor-4-(N-methyl-N-methoxycarbamoylamino)-phenyl, 2-Methyl-4-(N-methyl-N-methoxycarbamoylamino)-phenyl, 2-Chlor-5-(N-methyl-N-methoxycarbamoylamino)-phenyl, 2-Methyl-5-(N-methyl-N-methoxycarbamoylamino)-phenyl, 3-(N,N-Diethylcarbamoylamino)-phenyl, 4-(N,N-Diethylcarbamoylamino)-phenyl, 2,6-Dichlor-4-(N,N-Dimethylcarbamoylamino)-phenyl, 2,6-Dichlor-4-(N-methyl-N-methoxycarbamoylamino)phenyl, 2-Acetylamino-phenyl, 3-Acetylaminophenyl, 4-Acetylaminophenyl, 2-Propionylaminophenyl, 3-Propionylaminophenyl, 4-Propionylaminophenyl, 2-Pivaloylaminophenyl, 3-Pivaloylaminophenyl, 4-Pivaloylaminophenyl, 2-Chlor-4-acetylaminophenyl, 2-Chlor-4-pivaloylaminophenyl, 2-Chlor-4-propionylaminophenyl, 2-Chlor-5-propionylaminophenyl, 2-Chlor-5-Acetylaminophenyl, 3-Cyclopropylcarbonylaminophenyl, 4-Cyclopropylcarbonylaminophenyl, 3-Cyclopentylcarbonylaminophenyl, 4-Cyclopentylcarbonylaminophenyl, 2-Chlor-4-cyclopropylcarbonylaminophenyl, 2-Chlor-5-cyclopropylcarbonylaminophenyl, 2-Methyl-4-cyclopropylcarbonylaminophenyl, 2-Methyl-5-cyclopropylcarbonylaminophenyl, 2-Cyclohexylcarbonylaminophenyl, 3-Cyclohexylcarbonylaminophenyl, 4-Cyclohexylcarbonylaminophenyl, 2-Chlor-4-Cyclohexylcarbonylaminophenyl, 2-Fluor-4-cyclopropylcarbonylaminophenyl, 2-Fluor-5-cyclopropylcarbonylaminophenyl, 2-Fluor-5-cyclohexylcarbonylaminophenyl, 2,6-Dichlor-4-cyclopropylcarbonylaminophenyl, 2,6-Dichlor-4-cyclohexylcarbonylaminophenyl, 2-Methoxycarbonylaminophenyl, 2-i-Propoxycarbonylaminophenyl, 2-Ethoxycarbonylaminophenyl, 3-Methoxycarbonylaminophenyl, 3-Ethoxycarbonylaminophenyl, 4-Ethoxycarbonylaminophenyl, 4-n-Butoxycarbonylaminophenyl, 4-t-Butoxycarbonylaminophenyl, 2-Chlor-4-Ethoxycarbonylaminophenyl, 2-Chlor-5-ethoxycarbonylaminophenyl, 2-Brom-5-ethoxycarbonylaminophenyl, 2-Fluor-5-ethoxycarbonylaminophenyl.

Zur Herstellung der Isoharnstoffe der Formel I sind mehrere Synthesewege möglich:

1. Man erhält die Isoharnstoffe der Formel I

a) durch Umsetzung von N-Arylchlorformimidsäurearylestern der Formel

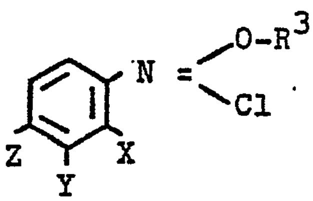

(II),

in der X, Y, Z und $R^3$ die obengenannten Bedeutung haben, mit einem Amin der Formel III

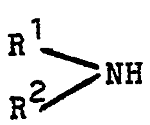

(III),

in der $R^1$ und $R^2$ die obengenannten Bedeutungen haben oder

b) durch Umsetzung von Chlorformamidinen der Formel IV

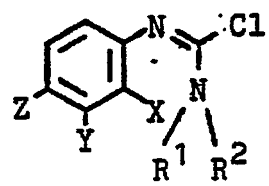

(IV),

in der X, Y, Z, $R^1$ und $R^2$ die obengenannten Bedeutungen haben, mit einem Phenol der Formel (V)

$R^3OH$ (V),

in der $R^3$ die obengenannten Bedeutungen hat, in Gegenwart eines säurebindenden Mittels.

Die N-Arylchlorformimidsäurearylester der Formel II lassen sich durch Umsetzung von Arylisocyaniddichloriden mit Phenolaten, beispielsweise Natriumphenolaten der Formel $NaOR^3$ herstellen (Houben-Weyl, Methoden der org. Chemie, Bd. E 4, S. 544 ff (1983)).

Die Chlorformamidine der Formel IV können durch Umsetzung von trisubstituierten Harnstoffen bzw. Thioharnstoffen mit Chlorierungsmitteln, wie $PCl_5$ bwz, $COCl_2$ erhalten werden (Houben-Weyl, Methoden der org. Chemie, Bd. E 4, S. 555 ff (1983)). Wenn $R^1$ und $R^2$ in Formel IV der Chlorformamidine Alkyl, vorzugsweise Methyl, bedeuten, kann es vorteilhaft sein die Chlorformamidine ausgehend von Phosgeniminiumchlorid entsprechend folgendem Reaktionsschema herzustellen:

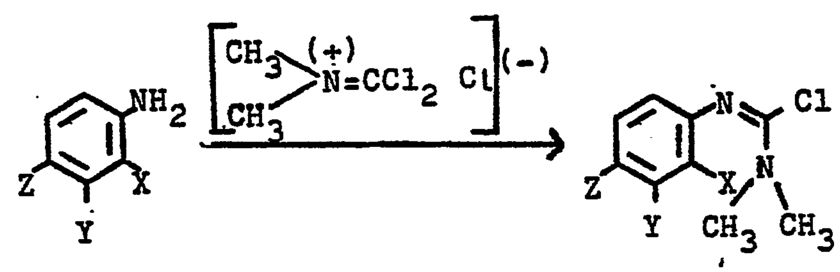

(Houben-Weyl, Methoden der org. Chemie, Bd. E 4, Seite 558 (1983)).

Die technische Verfügbarkeit bestimmt von Fall zu Fall das ausgewählte Verfahren. Besonders vorteilhaft ist die Herstellung der Isoharnstoffe der Formel I aus Chlorformamidinen der Formel IV, die aus trisubstituierten Harnstoffderivaten der Formel

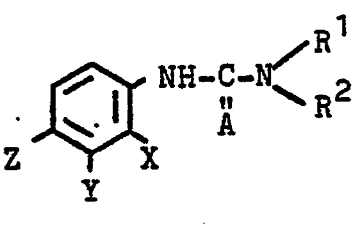

(VI),

in der A Sauerstoff oder Schwefel bedeutet und X, Y, Z, $R^1$ und $R^2$ die obengenannten Bedeutungen haben, durch Umsetzung mit Chlorierungsmitteln erhalten werden. Hierbei werden die Chlorformamidine IV bevorzugt im Verhältnis 1 bis 1 : 1,4 mit den Phenolen der Formel $R^3$-OH umgesetzt.

Die Umsetzungen zur Herstellung der Isoharnstoffe der Formel I werden zweckmäßigerweise in gegenüber den Reaktionsteilnehmern inerten Lösungs- oder Verdünnungsmitteln ausgeführt. Hierzu sind beispielsweise geeignet: aliphatische und aromatische, gegebenenfalls chlorierte Kohlenwasserstoffe, wie Petrolether, Benzol, Toluol, Xylol, Benzin, Dichlormethan, Chloroform, Tetrachlormethan, 1,2-Dichlorethan, Chlorbenzol, Ether wie Diethyl- und Di-n-butylether, Methyl-tert.-butylether, Tetrahydrofuran, Dioxan; Ketone, beispielsweise Aceton, Methylethylketon, Methylisopropylketon; Nitrile, wie Acetonitril und Propionitril, ferner Amide wie N-Methylpyrrolidon, Dimethylformamid. Auch Gemische dieser Lösungs- oder Verdünnungsmittel können verwendet werden.

Als säurebindende Mittel können die bei der Acylierung von Hydroxyverbindungen üblichen basischen Mittel Verwendung finden. Als besonders geeignet erweisen sich Alkalimetallcarbonate oder -alkoholate, wie Natrium- und Kaliumcarbonat and Kalium-t-butylat, ferner aliphatische, aromatische und heterocyclische Amine, z. B. Triethylamin, Piperidin, Dimethylanilin, Dimethylbenzylamin und Pyridin.

Statt in Gegenwart eines Säureacceptors zu arbeiten, ist es ebenso gut möglich, zunächst die Salze, etwa die Alkalimetall-, Erdalkalimetall -oder Ammoniumsalze der Phenole der Formel $R^3OH$ herzustellen und diese anschließend mit der Verbindung der Formel III weiter umzusetzen.

Die Verfahren zur Herstellung der Isoharnstoffe der Formel I können kontinuierlich oder diskontinuierlich, drucklos oder unter Druck, durchgeführt werden, der Einfachheit halber arbeitet man vorzugsweise bei Atmosphärendruck.

Die Reaktionstemperatur ist innerhalb eines größeren Bereiches variierbar. Im allgemeinen arbeitet man in einem Temperaturbereich von ungefähr 0 bis 120°C, vorzugsweise im Bereich zwischen 20 und 50 °C. Da die Reaktion in einigen Fällen exotherm verläuft, kann eine Außenkühlung zu Beginn der Reaktion von Vorteil sein.

Zur Vervollständigung der Reaktion wird das Reaktionsgemisch noch 15 Minuten bis 24 Stunden, vorzugsweise 1 Stunde bis 5 Stunden, gerührt. Danach wird je nach Lösungsmittel direkt mit Wasser gewaschen oder das Solvens wird abdestilliert, der Rückstand in einem mit Wasser nicht mischbaren Lösungsmittel und Wasser behandelt. In jedem Fall wird nach Phasentrennung die organische Schicht eingeengt, das als Rückstand erhaltene Produkt kann, wenn notwendig, durch Umkristallisation oder Säulenchromatographie gereinigt werden.

Die folgenden Beispiele erläutern die Herstellung der Isoharnstoffe der Formel I:

Beispiel 1

10,4 g (0,05 Mol) Phosphorpentachlorid und 11,6 g (0,05 Mol) N,N-Dimethyl-N'-(3,4-dichlorphenyl)-harnstoff werden in 75 ml Toluol 3 Stunden unter Rückfluß gekocht. Nach Abdestillieren der flüchtigen Anteile bei 0,1 mm Hg. erhält man 12,6 g N,N-Dimethyl-N'-(3,4-dichlorphenyl)-chlorformamidin in Form eines Öls, $n_D^{25}$ : 1,6133

| | C | H | N | Cl |
|---|---|---|---|---|
| Ber. | 42,97 | 3,7 | 11,1 | 42,28 |
| Gef. | 42,7 | 3,7 | 10,6 | 42,7 |

Zu 9,8 g (0,05 Mol) 2-(4-Hydroxy-phenoxy)-propionsäuremethylester in 75 ml trockenem Dimethylformamid gibt man 12,6 g (0,05 Mol) N,N-Dimethyl-N'-(3,4-dichlorphenyl)-chlorformamidin und anschließend portionsweise bei 10 bis 20°C 5,6 g (0,05 Mol) Kalium-t-butylat, anschließend wird 2 Stunden bei 50°C gerührt, danach das Lösungsmittel bei 10 Torr abdestilliert; der Rückstand wird dann mit Methylenchlorid/Wasser behandelt. Beim Einengen der über Natriumsulfat getrockneten Methylenchloridlösung erhält man einen öligen Rückstand der auf einer Kieselgelsäule mit Toluol/Essigester 1 : 1 gereinigt wird. Es verbleiben 14,6 g N,N-Dimethyl-N'-(3,4-dichlorphenyl-0-[4-(1-methoxycarbonyl-)ethyl-1-oxy-]-phenyl-isoharnstoff;
$n_D^{25}$ = 1,5732.

| | C | H | N | Cl |
|---|---|---|---|---|
| Ber. | 55,5 | 4,9 | 6,81 | 17,2 |
| Gef. | 55,8 | 5,0 | 6,4 | 17,0 |

Analog werden folgende Isoharnstoffe der Formel I hergestellt:

| | $R^1$ | $R^2$ | $R^3$ | X | Y | Z | $Fp[°C]/n^{20}_D$ |
|---|---|---|---|---|---|---|---|
| 1 | $CH_3$ | $CH_3$ | $4-[(CH_3)_2NCO-NH]C_6H_4$ | H | Cl | Cl | 137 - 139 |
| 2 | $CH_3$ | $OCH_3$ | $4-[(CH_3)_2NCO-NH]C_6H_4$ | H | Cl | Cl | 140 - 142 |
| 3 | $CH_3$ | $OCH_3$ | $4-[(CH_3)_2NCO-NH]C_6H_4$ | H | Cl | $CH_3$ | 121 - 122 |
| 4 | $CH_3$ | $OCH_3$ | $4-[(CH_3)_2NCO-NH]C_6H_4$ | H | $CF_3$ | H | |
| 5 | $CH_3$ | $OCH_3$ | $4-[(CH_3)_2NCO-NH]C_6H_4$ | H | H | F | |
| 6 | $CH_3$ | $OCH_3$ | $4-[(CH_3)_2NCO-NH]C_6H_4$ | H | H | H | 147 |
| 7 | $CH_3$ | $CH_3$ | $4-[(CH_3)_2NCO-NH]C_6H_4$ | H | H | 4-Chlorphenoxy | |
| 8 | $CH_3$ | $CH_3$ | $4-[\,^{CH_3}_{CH_3O}{>}N-CO-NH]C_6H_4$ | H | Cl | Cl | 104 - 105 |
| 9 | $CH_3$ | $OCH_3$ | $4-[\,^{CH_3}_{CH_3O}{>}N-CO-NH]C_6H_4$ | H | Cl | Cl | 93 - 96 |
| 10 | $CH_3$ | $OCH_3$ | $4-[\,^{CH_3}_{CH_3O}{>}N-CO-NH]C_6H_4$ | H | Cl | $CH_3$ | |
| 11 | $CH_3$ | $CH_3$ | $4-[\,^{CH_3}_{CH_3O}{>}N-CO-NH]C_6H_4$ | H | $CF_3$ | H | 122 - 124 |
| 12 | $CH_3$ | $OCH_3$ | $4-[\,^{CH_3}_{CH_3O}{>}N-CO-NH]C_6H_4$ | H | H | H | 1,5780 |
| 13 | $CH_3$ | $OCH_3$ | $4-[\,^{CH_3}_{CH_3O}{>}N-CO-NH]C_6H_4$ | H | H | 2-(4-Methyl-phenyl)-ethoxy | 1,5880 |
| 14 | $CH_3$ | $CH_3$ | $4-C_2H_5CONHC_6H_4$ | H | Cl | Cl | 1,6008 |
| 15 | $CH_3$ | $OCH_3$ | $4-C_2H_5CONHC_6H_4$ | H | Cl | Cl | 1,5938 |
| 16 | $CH_3$ | $OCH_3$ | $4-C_2H_5CONHC_6H_4$ | H | Cl | $CH_3$ | 1,5770 |

| | $R^1$ | $R^2$ | $R^3$ | X | Y | Z | Fp[°C]/$n^{20}_D$ |
|---|---|---|---|---|---|---|---|
| 17 | $CH_3$ | $CH_3$ | $4-C_2H_5OCONHC_6H_4$ | H | H | $i-C_3H_7$ | |
| 18 | $CH_3$ | $CH_3$ | $4-C_2H_5OCONHC_6H_4$ | F | H | H | |
| 19 | $CH_3$ | $CH_3$ | $4-C_2H_5OCONHC_6H_4$ | H | H | 4-Chlorphenoxy | |
| 20 | $CH_3$ | $CH_3$ | $4-C_2H_5OCONH-C_6H_4$ | H | Cl | Cl | 98 - 100 |
| 21 | $CH_3$ | $OCH_3$ | $4-C_2H_5OCONH-C_6H_4$ | H | Cl | Cl | 96 - 97 |
| 22 | $CH_3$ | $OCH_3$ | $4-C_2H_5OCONH-C_6H_4$ | H | Cl | $CH_3$ | 1,5780 |
| 23 | $CH_3$ | $OCH_3$ | $4-C_2H_5OCONH-C_6H_4$ | H | $CF_3$ | H | |
| 24 | $CH_3$ | $CH_3$ | $4-C_2H_5OCONH-C_6H_4$ | H | H | 2-(4-Methyl-phenyl)-ethoxy | |
| 25 | $CH_3$ | $CH_3$ | $4-C_2H_5OCONH-C_6H_4$ | H | H | $i-C_3H_7$ | |
| 26 | $CH_3$ | $CH_3$ | $4-[CH_3OCO-CH(CH_3)O]-C_6H_4$ | H | Cl | Cl | 1,5732 |
| 27 | $CH_3$ | $OCH_3$ | $4-[CH_3OCO-CH(CH_3)O]-C_6H_4$ | H | Cl | $CH_3$ | 1,5582 |
| 28 | $CH_3$ | $CH_3$ | $4-[CH_3OCO-CH(CH_3)O]-C_6H_4$ | H | $CF_3$ | H | 1,5228 |
| 29 | $CH_3$ | $CH_3$ | $4-[CH_3OCO-CH(CH_3)O]-C_6H_4$ | H | H | $i-C_3H_7$ | |
| 30 | $CH_3$ | $CH_3$ | $4-[CH_3OCO-CH(CH_3)O]-C_6H_4$ | H | F | H | |
| 31 | $CH_3$ | $CH_3$ | $4-[CH_3OCO-CH(CH_3)O]-C_6H_4$ | H | H | 4-Chlorphenoxy | |
| 32 | $CH_3$ | $CH_3$ | $3-CH_3O-C_6H_4$ | H | Cl | Cl | 1,6080 |
| 33 | $CH_3$ | $CH_3$ | $4-CH_3O-C_6H_4$ | H | Cl | Cl | 72 - 74 |
| 34 | $CH_3$ | $CH_3$ | $4-t-C_4H_9O-C_6H_4$ | H | Cl | Cl | |
| 35 | $CH_3$ | $CH_3$ | $4-t-C_4H_9O-C_6H_4$ | H | $CF_3$ | H | |

| | $R^1$ | $R^2$ | $R^3$ | X | Y | Z | Fp[°C]/$n^{20}_D$ |
|---|---|---|---|---|---|---|---|
| 36 | $CH_3$ | $CH_3$ | 4-t-$C_4H_9O$-$C_6H_4$ | H | H | i-$C_3H_7$ | |
| 37 | $CH_3$ | $CH_3$ | 3-$CH_3OCONH$-$C_6H_4$ | H | Cl | Cl | 1,5985 |
| 38 | $CH_3$ | $OCH_3$ | 3-$CH_3OCONH$-$C_6H_4$ | H | Cl | Cl | 109 |
| 39 | $CH_3$ | $OCH_3$ | 3-$CH_3OCONH$-$C_6H_4$ | H | $CF_3$ | H | 1,5340 |
| 40 | $CH_3$ | $OCH_3$ | 3-$(CH_3)_2NCONH$-$C_6H_4$ | H | Cl | Cl | 115 - 116 |
| 41 | $CH_3$ | $OCH_3$ | 3-$(CH_3)_2NCONH$-$C_6H_4$ | H | $CF_3$ | H | 1,5360 |
| 42 | $CH_3$ | $CH_3$ | 3-$(CH_3)_2NCONH$-$C_6H_4$ | H | H | 4-Chlorphenoxy | |
| 43 | $CH_3$ | $CH_3$ | 3-$(CH_3)_2NCONH$-$C_6H_4$ | H | Cl | $CH_3$ | |
| 44 | $CH_3$ | $CH_3$ | 3-$(CH_3)_2NCONH$-$C_6H_4$ | H | H | F | |
| 45 | $CH_3$ | $CH_3$ | 2-$CH_3$-5-[$(CH_3)_2N$-CONH]-$C_6H_3$ | H | Cl | Cl | 132 - 134 |
| 46 | $CH_3$ | $OCH_3$ | 2-$CH_3$-5-[$(CH_3)_2N$-CONH]-$C_6H_3$ | H | Cl | Cl | 1,5843 |
| 47 | $CH_3$ | $OCH_3$ | 2-$CH_3$-5-[$(CH_3)_2N$-CONH]-$C_6H_3$ | H | $CF_3$ | H | 133 - 135 |
| 48 | $CH_3$ | $OCH_3$ | 2-$CH_3$-5-[$(CH_3)_2N$-CONH]-$C_6H_3$ | H | H | H | |
| 49 | $CH_3$ | $CH_3$ | 3-Cyclo-$C_3H_5$-CONH-$C_6H_4$ | H | Cl | Cl | 54 - 56 |
| 50 | $CH_3$ | $CH_3$ | 2-Cl-4-[$CH_3N(OCH_3)CONH$]-$C_6H_3$ | H | Cl | Cl | 1,5915 |
| 51 | $CH_3$ | $OCH_3$ | 2-Cl-4-[$CH_3N(OCH_3)CONH$]-$C_6H_3$ | H | Cl | Cl | 1,5830 |
| 52 | $CH_3$ | $OCH_3$ | 2-Cl-4-[$CH_3N(OCH_3)CONH$]-$C_6H_3$ | H | H | H | 1,5675 |
| 53 | $CH_3$ | $CH_3$ | 2-Cl-4-[$CH_3N(OCH_3)CONH$]-$C_6H_3$ | H | $CF_3$ | H | |
| 54 | $CH_3$ | $OCH_3$ | 2-Cl-4-[$CH_3N(OCH_3)CONH$]-$C_6H_3$ | H | $CF_3$ | H | |
| 55 | $CH_3$ | $CH_3$ | 2-$CH_3$-5-[$CH_3N(OCH_3)CONH$]-$C_6H_3$ | H | Cl | Cl | 127 - 129 |

| | $R^1$ | $R^2$ | $R^3$ | X | Y | Z | Fp[°C]/$n^{20}_D$ |
|---|---|---|---|---|---|---|---|
| 56 | $CH_3$ | $OCH_3$ | 2-$CH_3$-5-[$CH_3N(OCH_3)CONH$]-$C_6H_3$ | H | Cl | Cl | 1,5740 |
| 57 | $CH_3$ | $OCH_3$ | 2-$CH_3$-5-[$CH_3N(OCH_3)CONH$]-$C_6H_3$ | H | $CF_3$ | H | |
| 58 | $CH_3$ | $CH_3$ | 2,6-$Cl_2$-4-[$CH_3N(OCH_3)CONH$]-$C_6H_2$ | H | Cl | Cl | 1,5895 |
| 59 | $CH_3$ | $CH_3$ | 2,6-$Cl_2$-4-[$CH_3N(OCH_3)CONH$]-$C_6H_2$ | H | $CF_3$ | H | |
| 60 | $CH_3$ | $CH_3$ | 2,6-$Cl_2$-4-[$CH_3N(OCH_3)CONH$]-$C_6H_2$ | H | H | H | |
| 61 | $CH_3$ | $OCH_3$ | 2-Cl-4-[$(CH_3)_2NCONH$]-$C_6H_3$ | H | Cl | Cl | 116 - 118 |
| 62 | $CH_3$ | $CH_3$ | $-CH_2-C_6H_5$ | H | Cl | Cl | |

Die Isoharnstoffe der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, z.B. Methanol, Ethanol, Propanol, Butanol, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeemerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gew.-%, vorzugsweise zwischen 0,5 bis 90 Gew.-% Wirkstoff.

Beispiele für Formulierungen sind:

I. Man vermischt 90 Gew.-Teile des Wirkstoffs Nr. 8 mit 10 Gew.-Teilen N-Methyl-pyrrolidon und erhält eine Lösung, die zur Anwendung in Form kleinster Tropfen geeignet ist.

II. 10 Gew.-Teile des Wirkstoffs Nr. 28 werden mit einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecyl-benzol-sulfonsäure und 2 Gew.-Teile des Anlagerungsproduktes von 40 Mol Ethylenoxid an 1 Mol Ricinusöls besteht.

III. 20 Gew.-Teile des Wirkstoffs Nr. 39 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

IV. 20 Gewichtsteile des Wirkstoffs Nr. 50 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält an eine wäßrige Dispersion, die 0,02 Gew.-% des Wirkstoffs enthält.

V. 80 Gew.-Teile des Wirkstoffs Nr. 40 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

VI. 5 Gew.-Teile des Wirkstoffs Nr. 41 werden mit 95 Gew.-Teilen feinteiligem Kaolin vermischt. Man erhält auf diese Weise ein Stäubemittel, das 5 Gew.-% des Wirkstoffs enthält.

VII. 30 Gew.-Teile des Wirkstoffs Nr. 42 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gew.-Teile des Wirkstoffs Nr. 15 werden mit 2 Teien Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabilie ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-driected, lay-by).

Die Aufwandmengen an Wirkstoff betragen je nach Bekämpfungsziel, Jahreszeit, Zielpflanzen und Wachstumsstadium 0,05 bis 5,0, vorzugsweise 0,25 bis 3,0 kg/ha.

Die herbizide Wirkung der Harnstoffderivate der Formel I auf das Wachstum von Testpflanzen wird durch folgende Gewächshausversuche gezeigt.

Als Kulturgefäße dienen Plastikblumentöpfe mit 300 $cm^3$ Inhalt und lehmigem Sand mit etwa 3,0 % Humus als Substrat. Bei Soja wird etwas Torfmull zugesetzt, um einen besseren Stand zu erzielen. Die Samen der Testpflanzen werden nach Arten getrennt flach eingesät.

Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmengen betragen 3,0 und 2,0 kg Wirkstoff/ha. Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zweck der Nachauflaufbehandlung zieht man die Testpflanzen je nach Wuchsform erst bis zu einer Wuchshöhe von 3 bis 15 cm an und behandelt sie danach. Es werden direkt gesäte und in den gleichen Gefäßen aufgewachsene Pflanzen ausgewählt, oder sie werden erst als Keimpflanzen getrennt angezogen. Die Aufwandmengen für die Nachauflaufbehandlung variieren je nach Wirkstoff von 0,125 bis 3,0 kg/ha. Eine Abdeckung unterblieb bei der Nachauflaufbehandlung.

Die Versuchsgefäße werden im Gewächshaus aufgestellt, wobei für wärmeliebende Arten wärmere Bereiche (20 bis 35°C) und für solche gemäßigter Klimate 10 bis 20°C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlig Zerstörung zumindest der oberirdischen Teile.

Die in den Gewächshausversuchen verwendeten Pflanzen setzen sich aus folgenden Arten zusammen:

Amaranthus spp. (Fuchsschwanzarten),
Amaranthus retroflexus (zurückgekrümmter Fuchsschwanz),
Arachis hypogaea (Erdnuß),
Cassia spp.,
Cassia tora,
Chenopodium album (weißer Gänsefuß),
Desmodium tortuosum,
Galium aparine (Klettenlabkraut),
Glycine max (Sojabohnen),
Gossypim hirsutum (Baumwolle),
Helianthus annuus (Sonnenblume),
Ipomoea spp. (Prunktwindearten),
Lamium amplexicaule (stengelumfassende Taubnessel),
Lolium multiflorum (ital. Raygras),
Mercurialis annua (einjähriges Bingelkraut),
Sesbania exaltata (Turibaum),
Sida spinosa,
Sinapis alba (weißer Senf),
Solanum nigrum (schwarzer Nachtschatten)
Sorghum halepense (Sudangras),

Triticum aestivum (Weizen).
Abutiolon theophrasti (velvetleaf)
Avena fatua (wild oats)
Digitaria sanguinalis (crabgrass)
Viola tricolor (violet)

Bei Vorauflaufanwendung von 3,0 kg Wirkstoff/ha zeigen beispielsweise die Verbindungen Nr. 3, 40, 11, 41, 1, 62, 46 und 38 eine beachtliche herbizide Wirkung. Bei dieser Anwendungsmethode bekämpft beispielsweise die Verbindung Nr. 1 mit einer Aufwandmenge von 2,0 kg Wirkstoff/ha unerwünschte Pflanzen selektiv in Sojabohnen und Weizen.

Tabelle 1 - Herbizide Aktivität bei Vorauflaufanwendung von 3,0 kg/ha im Gewächshaus

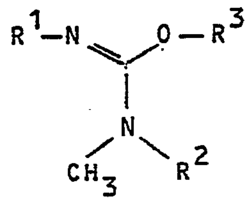

| Beisp. Nr. | $R^1$ | $R^2$ | $R^3$ | Testpflanzen und Schädigung % Lolium multiflorum | Sinapis alba |
|---|---|---|---|---|---|
| 3 | 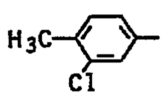 | $-OCH_3$ | 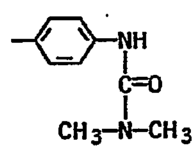 | 90 | 100 |
| 40 | 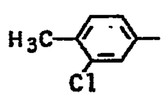 | $-OCH_3$ | 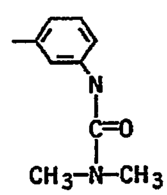 | 90 | 100 |
| 11 | 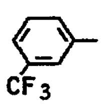 | $-CH_3$ | 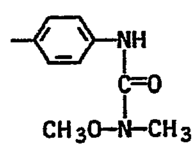 | 99 | 98 |
| 41 | 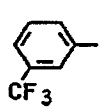 | $-OCH_3$ | 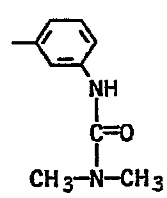 | 95 | 100 |
| 1 | 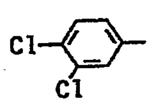 | $-CH_3$ | 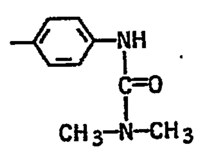 | 100 | 98 |

| Beisp. Nr. | $R^1$ | $R^2$ | $R^3$ | Testpflanzen und Schädigung % Lolium multiflorum | Sinapis alba |
|---|---|---|---|---|---|
| 62 | 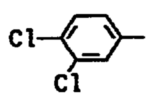 | $-CH_3$ | 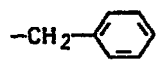 | 95 | 100 |
| 46 | 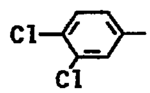 | $-OCH_3$ | 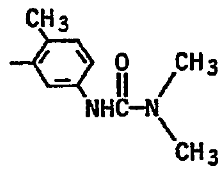 | 100 | 100 |
| 38 | 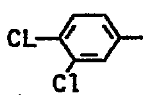 | $-OCH_3$ | 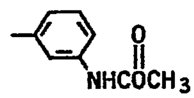 | 100 | 100 |

Bei Nachauflaufanwendung von 3,0 kg Wirkstoff/ha wirken die Verbindungen Nr. 9, 45, 46, 2, 11, 20, 21, 27, 13, 38, 37 herbizid gegen Ipomoea spp. und die Grasart Lolium multiflorum als beispielhaft angewandte Pflanzen. Verbindung Nr. 8 wirkt selektiv mit 1,0 kg Wirkstoff/ha in Kulturen, wie Erdnüsse und Baumwolle. Cassia spp. (C. tora, C. obtusiflora) läßt sich in Soja mit den Verbindungen Nr. 41, 39 und 38 bei einer Aufwandmenge von 1,0 bzw. 0,5 kg Wirkstoff/ha bekämpfen. Weiterhin bekämpfen beispielsweise die Verbindungen Nr. 1, 27, 49, 40, 46, 37 und 38 breitblättrige Pflanzen. Beispielhaft für die Wirkung gegen eine unerwünschte Grasart (Sorghum halepense) mit Selektivität in Baumwolle sind die Verbindungen Nr. 15, 14 und 40 bei einer Aufwandmenge von 1,0 kg Wirkstoff/ha. Die Verbindungen Nr. 50, 58, 55, 56, 33 wirken mit 0,5 kg aktiver Substanz pro Hektar und Nachauflaufanwendung gegen breitblättrige unerwünschte Pflanzen. Selektive herbizide Wirkung hat Verbindung Nr. 51 mit 0,5 kg Wirkstoff/ha bei Nachauflaufanwendung.

Tabelle 2 – Selektive Bekämpfung von unerwünschten breitblättrigen Pflanzen bei Vorauflaufanwendung von 2 kg/ha der Verbindung Nr. 1 im Gewächshaus

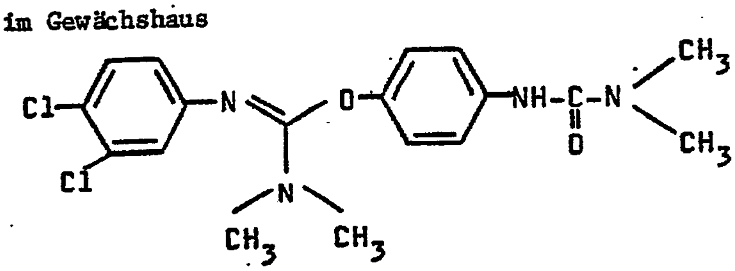

| Testpflanzen | Schädigung % |
|---|---|
| Glycine max | 10 |
| Triticum aestivum | 0 |
| Amaranthus retroflexus | 100 |
| Chenopodium album | 98 |
| Sinapis alba | 90 |
| Solanum nigrum | 98 |

Tabelle 3 - Herbizide Aktivität einiger Beispielsverbindungen bei Nachauflaufanwendung von 3,0 kg/ha im Gewächshaus

| Beisp. Nr. | $R^1$ | $R^2$ | $R^3$ | Testpflanzen und Schädigung % Ipomoea spp. | Lolium multiflorum |
|---|---|---|---|---|---|
| 9 | 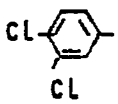 | $OCH_3$ | 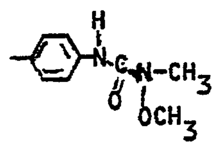 | 100 | 100 |
| 45 | 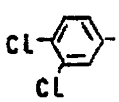 | $CH_3$ | 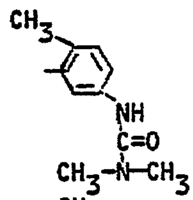 | 95 | 98 |
| 46 | 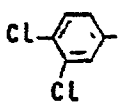 | $OCH_3$ | 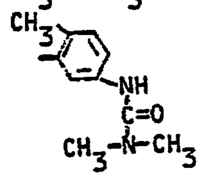 | 100 | 100 |
| 2 | 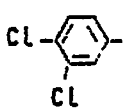 | $OCH_3$ | 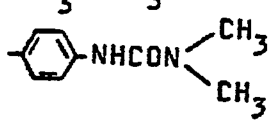 | 100 | 100 |
| 11 | 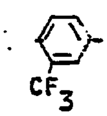 | $CH_3$ | 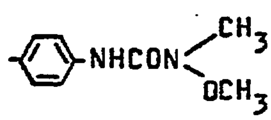 | 100 | 100 |
| 20 | 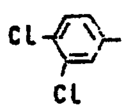 | $CH_3$ | 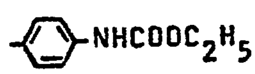 | 100 | 100 |
| 21 | 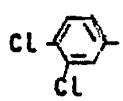 | $OCH_3$ | 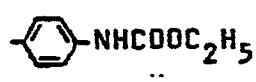 | 100 | 98 |
| 27 | 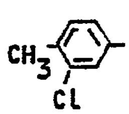 | $OCH_3$ | 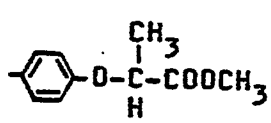 | 95 | 100 |

| Beisp. Nr. | $R^1$ | $R^2$ | $R^3$ | Testpflanzen und Schädigung % Ipomoea spp. | Lolium multiflorum |
|---|---|---|---|---|---|
| 13 | 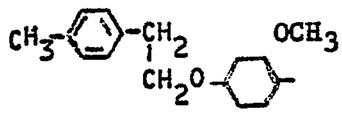 | $OCH_3$ | 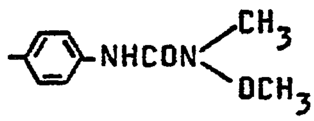 | 100 | 95 |
| 38 | 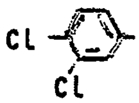 | $OCH_3$ | 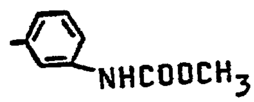 | 100 | 100 |
| 37 | 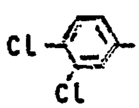 | $CH_3$ | 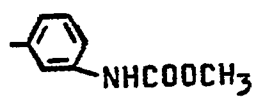 | 100 | 100 |

Tabelle 4 - Selektive Bekämpfung breitblättriger unerwünschter Pflanzen bei Nachauflaufanwendung von 1 kg/ha der Verbindung Nr. 8 Gewächshaus

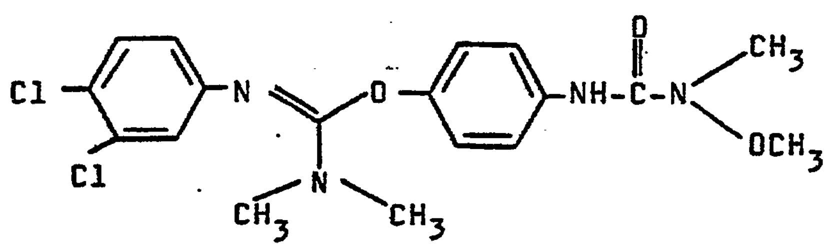

| Testpflanzen | Schädigung % |
|---|---|
| Arachis hypogaea | 5 |
| Gossypium hirsutum | 9 |
| Amaranthus spp. | 100 |
| Cassia tora | 95 |
| Ipomoea spp. | 89 |
| Chenopodium album | 100 |
| Desmodium tortuosum | 100 |
| Sesbania exaltata | 100 |
| Sida spinosa | 89 |

Tabelle 5 - Beispiel zur selektriven Bekämpfung breitblättriger Unkrauter bei Nachauflaufanwendung von 1 kg/ha der Verbindung Nr. 27 im Gewächshaus

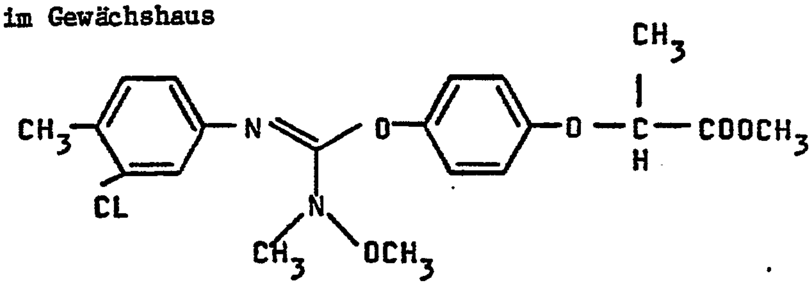

| Testpflanzen | Schädigung % |
|---|---|
| Gossypium hirsutum | 0 |
| Helianthus annuus | 10 |
| Iriticum aestivum | 10 |
| Chenopodium album | 100 |
| Lamium amplexicaule | 100 |

Tabelle 6 - Beispiel zur Bekämpfung breitblättriger Unkräuter in Erdnüssen im Nachaufläufverfahren von 1 kg/ha der Verbindung Nr. 49

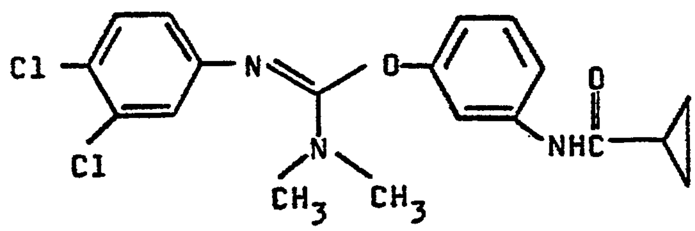

| Testpflanzen | Schädigung % |
|---|---|
| Arachis hypogaea | 0 |
| Desmodium tortuosum | 100 |
| Lamium amplexicaule | 100 |
| Mercurialis annua | 100 |
| Sesbania exaltata | 100 |

Tabelle 7 - Selektive Bekämpfung von Cassia spp. in Soja bei Nachauflaufanwendung von 1 kg/ha der Verbindung Nr. 39 im Gewächshaus

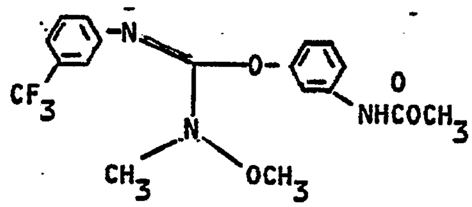

| Testpflanzen und Schädigung % | |
|---|---|
| Glycine max | Cassia spp. |
| 0 | 100 |

Tabelle 8 - Bekämpfung breitblättriger unerwünschter Pflanzen in Sonnenblumen mit 1 kg/ha der Beispielsverbindungen bei Nachauflaufanwendung im Gewächshaus

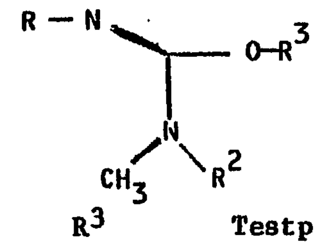

| Beisp. Nr. | R | $R^2$ | $R^3$ | Testpflanzen und Schädigung % Helianthus annuus | Chenopodium album | Galium aparine |
|---|---|---|---|---|---|---|
| 37 | 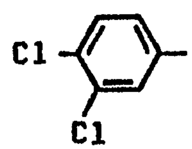 | $CH_3$ | 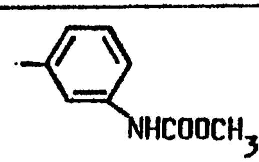 | 10 | 100 | |
| 40 | 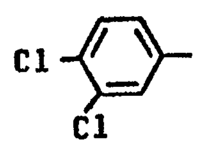 | $OCH_3$ | 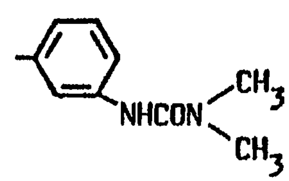 | 10 | 100 | |

Tabelle 9 - Selektive Bekämpfung von breitblättrigen Unkräutern in Baumwolle bei Nachauflaufanwendung von 1 kg/ha a. S. im Gewächshaus

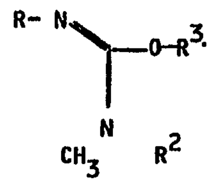

| Beisp. Nr. | R | $R^2$ | | Testpflanzen und Schädigung % Gossypium hirsutum | Amaranthus retroflexus | Cassia spp. |
|---|---|---|---|---|---|---|
| 46 | 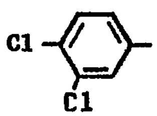 | $OCH_3$ | 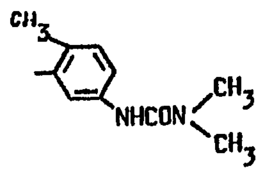 | 10 | 100 | 100 |
| 37 | 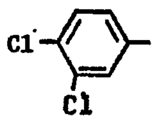 | $CH_3$ | 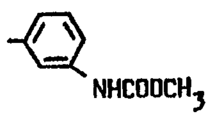 | 0 | 100 | 100 |
| 38 | 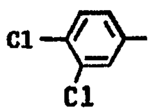 | $OCH_3$ | 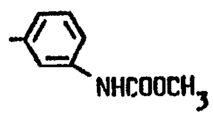 | 0 | 100 | 100 |

Tabelle 10 - Bekämpfung von Sorghum halepense als grasartige Beispielspflanze bei Nachauflaufanwendung von 1 kg/ha a. S. im Gewächshaus

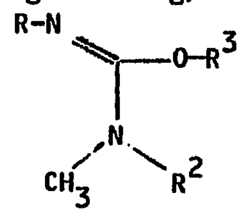

| Beisp. Nr. | R | $R^2$ | $R^3$ | Testpflanzen und Schädigung % Gossypium hirsutum | Sorghum halepense |
|---|---|---|---|---|---|
| 15 | 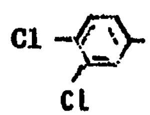 | $OCH_3$ | 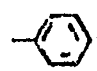 -NHCOC$_2$H$_5$ | 5 | 95 |
| 14 | 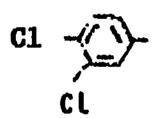 | $CH_3$ | 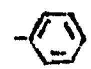 -NHCOC$_2$H$_5$ | 0 | 100 |
| 40 | 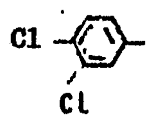 | $OCH_3$ | 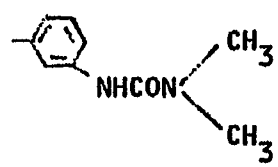 | 0 | 100 |

Tabelle 11 - Herbizide Aktivität verschiedener Beispielsverbindungen bei Nachauflaufanwendung von 0,5 kg/ha a.S. im Gewächshaus

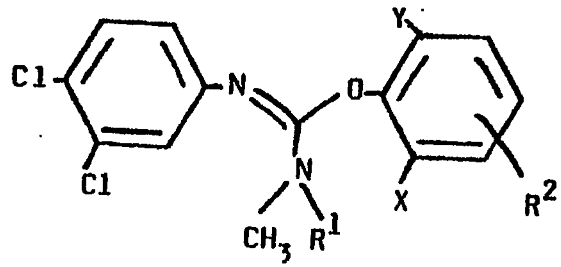

| Wirkstoff-Nr. | $R^1$ | X | Y | $R^2$ | Stellung | Testpflanzen und Schädigung % Cassia tora | Galium aparine | Ipomoea spp. | Sinapis alba | Solanum nigrum |
|---|---|---|---|---|---|---|---|---|---|---|
| 50 | $CH_3$ | Cl | H | 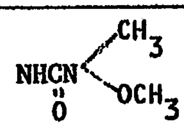 | 4 | 100 | 90 | 100 | 80 | 100 |
| 58 | $CH_3$ | Cl | Cl | 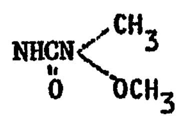 | 4 | 100 | 80 | 100 | 100 | 85 |
| 55 | $CH_3$ | H | $CH_3$ | 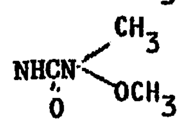 | 3 | 100 | 90 | 100 | 90 | 100 |
| 56 | $OCH_3$ | H | $CH_3$ | 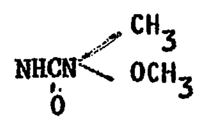 | 3 | 95 | 85 | 100 | 100 | 100 |
| 33 | $CH_3$ | H | H | $-OCH_3$ | 4 | 100 | 100 | 80 | 90 | 90 |

Tabelle 12 - Bekämpfung breitblättriger Unkräuter in Soja bei Nachauflaufanwendung von 0,5 kg/ha der Verbindung Nr. 51 im Gewächshaus

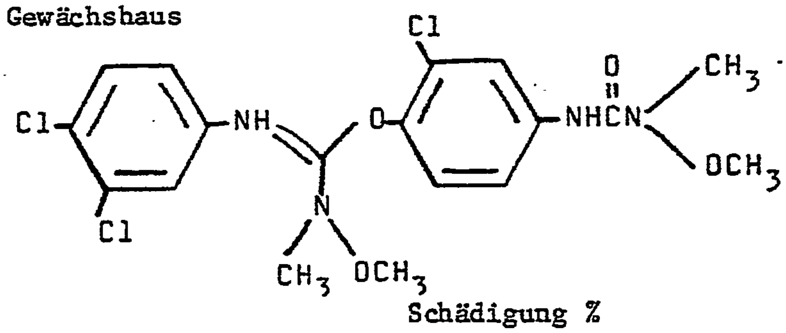

| Testpflanzen | Schädigung % |
|---|---|
| Glycine max | 10 |
| Abutilon | 80 |
| Amaranthus retroflexus | 100 |
| Cassia tora | 100 |
| Ipomoea spp. | 100 |
| Sinapis alba | 100 |

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgenden Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrasica | Kohlrübe |
| Brassica napus var. rapa | Weiße Rübe |
| Brassica rapa var, silvertris | Rüben |
| Camellia sinensis | Teestrauch |
| Carthamus tinctorius | Saflor - Färberdistel |
| Caria illioinensis | Pekannußbaum |
| Citrus limon | Zitrone |
| Citrus maxima | Pampelmuse |
| Citrus retriculata | Mandarine |
| Citrus sinensis | Apfelsine, Orange |
| Coffea arabica (Coffea canaphora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium virtifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |

| Botanischer Name | Deutscher Name |
|---|---|
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Petroseolinun crispum spp. tubersum | Wurzelpetersilie |
| Pica abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunsu persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sasmum indicum | Sesam |
| Solanum tuberosum | Kartoffel |
| Sorghum bicolor (s. vulgare) | Mohrenhirse |
| Spinacia oleracea | Spinat |
| Theobroma cacao | Kakaobaum |
| Trifolium pratense | Rotklee |

| Botanischer Name | Deutscher Name |
|---|---|
| Triticum aestivum | Weizen |
| Vaccinium corymbosum | Kulturheidelbeere |
| Vacinium vitis-idaea | Preißelbeere |
| Vicia faba | Pferdebohnen |
| Vigna sinensis (V. unguicultata) | Kuhbohne |
| Vitis vinifera | Weinrebe |
| Zea may | Mais (Unterblatt oder post-directed) |

Zur Verbreiterung des Wirkungsspektrums und zur Erzielung synergistischer Effekte können die neuen substituierten Isoharnstoffe sowohl unter sich als auch mit zahlreiche Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren und andere in Betracht. Eine Reihe von Wirkstoffen, welche zusammen mit den Isoharnstoffen der Formel I für verschiedenste Anwendungsbereiche sinnvolle Mischungen ergeben, werden beispielhaft aufgeführt:

3-(1-Methylethyl)-1H-2,1,3-benzothiadiazin-4(3H)-on-2,2-dioxid und Salze
3-(1-Methylethyl)-8-chlor-1H-2,1,3,-benzothiadiazin-4(3H)-on-2,2-dioxid und Salze
3-(1-Methylethyl)-8-fluor-1H-2,1,3-benzothiadiazin-4(3H)-on-2,2-dioxid und Salze
3-(1-Methylethyl)-8-methyl-1H-2,1,3-benzothiadiazin-4(3H)-on-2,2-dioxid und Salze
1-Methoxymethyl-3-(1-methylethyl)-2,1,3-benzothiadiazin-4(3H)-on-2,2-dioxid
1-Methoxymethyl-8-chlor-3-(1-methylethyl)-2,1,3-benzothiadiazin-4(3H)-on-2,2-dioxid
1-Methoxymethyl-8-fluor-3-(1-methylethyl)-2,1,3-benzothiadiazin-4(3H)-on-2,2-dioxid
1-Cyan-8-chlor-3-(1-methylethyl)-2,1,3-benzothiadiazin-4(3H)-on-2,2-dioxid
1-Cyan-8-fluor-3-(1-methylethyl)-2,1,3-benzothiadiazin-4(3H)-on-2,2-dioxid
1-Cyan-8-methyl-3-(1-methylethyl)-2,1,3-benzothiadiazin-4(3H)-on-2,2-dioxid
1-Azidomethyl-3-(1-methylethyl)-2,1,3-benzothiadiazin-4(3H)-on-2,2-dioxid
3-(1-Methylethyl)-1H-(pyridino-[3,2-e]2,1,3-thiadiazin-(4)-on-2,2-dioxid
1-Cyan-3-(1-methylethyl)-2,1,3-benzothiadiazin-4(3H)-on-2,2-dioxid
N-(1-Ethylpropyl)-2,6-dinitro-3,4-dimethylanilin
N-(1-Methylethyl)-N-ethyl-2,6-dinitro-4-trifluormethyl-anilin
N-n-Propyl-N-β-chlorethyl-2,6-dinitro-4-trifluormethyl-anilin
N-n-Propyl-N-cyclopropylmethyl-2,6-dinitro-4-trifluor-methyl-anilin
N,N-Di-n-propyl-2,6-dinitro-3-amino-4-trifluormethylanilin
N,N-Di-n-propyl-2,6-dinitro-4-methyl-anilin
N,N-Di-n-propyl-2,6-dinitro-4-methylsulfonyl-anilin
N,N-Di-n-propyl-2,6-dinitro-4-aminosulfonyl-anilin
N,N-Di-beta-chlorethyl-2,6-dinitro-4-methyl-anilin
N-Ethyly-N-(2-methylallyl)-2,6-dinitro-4-trifluormethyl-anilin
N-Methylcarbaminsäure-3,4-dichlorbenzylester
N-Methylcarbaminsäure-2,6-di-tert.butyl-4-methylphenyl-ester
N-Phenylcarbaminsäure-isopropylester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-ester
N-3,4-Dichlorphenylcarbaminsäure-methylester
N-(4-Amino-benzolsulfonyl)-carbaminsäure-methylester
O-(N-Phenylcarbamoyl)-propanonoxim
N-Ethyl-2-(phenylcarbamoyl)-oxypropionsäureamid
3'-N-Isopropyl-carbamoyloxy-propionanilid
Ethyl-N-[3-(N'-phenylcarbamoyloxy)-phenyl]-carbamat
Methyl-N-[3-(N'-methyl-N'-phenylcarbamoyloxy)-phenyl]-carbamat
Isopropyl-N-[3-(N'-ethyl-N'-phenylcarbamoyloxy)-phenyl]-carbamat
Methyl-N-[3-(N'-3-methylphenylcarbamoyloxy)-phenyl]-carbamat

Ethyl-N-[3-(N'-3-chlor-4-fluorphenylcarbamoyloxy)-phenyl]-carbamat
Ethyl-N-[3-(N'-3,4-difluorphenylcarbamoyloxy)-phenyl]-carbamat
N,N-Diethyl-thiolcarbaminsäure-p-chlorbenzylester
N,N-Di-n-propyl-thiolcarbaminsäure-ethylester
N,N-Di-n-propyl-thiolcarbaminsäure-n-propylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolyl-methylester
N,N-Di-isopropyl-thiolcarbaminsäure-3-ethyl-5-isoxazolyl-methylester
N,N-Di-sec.butyl-thiolcarbaminsäure-ethylester
N,N-Di-sec.butyl-thiolcarbaminsäure-benzylester
N-Ethyl-N-cyclohexyl-thiolcarbaminsäure-ethylester
N-Ethyl-N-bicyclo[2.2.1]heptyl-thiolcarbaminsäureethylester
S-Ethyl-hexahydro-1H-azepin-1-carbothiolat
S-Ethyl-3-methylhexahydro-1-H-azepin-1-carbothiolat
N-Ethyl-N-n-butyl-thiolcarbaminsäure-n-propylester
N,N-Dimethyl-dithiocarbaminsäure-2-chlorallylester
N-Methyl-dithiocarbaminsäure-Natriumsalz
Trichloressigsäure-Natriumsalz
Alpha,alpha-Dichlorpropionsäure-Natriumsalz
Alpha,alpha-Dichlorbuttersäure-Natriumsalz
Alpha,alpha,beta,beta-Tetrafluorpropionsäure-Natriumsalz
Alpha-Methyl-alpha,beta-dichlorpropionsäure-Natriumsalz
Alpha-Chlor-beta-(4-chlorphenyl)-propionsäure-methylester
Alpha,beta-Dichlor-beta-phenylpropionsäure-methylester
Benzamido-oxy-essigsäure
2,3,5-Trijodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephthalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureethylester
2-[4-(4'-Chlorphenoxy)-phenoxy]-propionsäureisobutylester
2-[4-(2',4'-Dichlorphenoxy)-phenoxy]-propionsäuremethylester
2-[4-(4'-Trifluormethylphenoxy)-phenoxy]-propionsäure-methylester
2-[4-(2'-Chlor-4'-trifluorphenoxy)-phenoxy]-propionsäureNatriumsalz
2-[4-(3',5'-Dichlorpyridyl-2-oxy)-phenoxy]-propionsäureNatriumsalz
2-(N-Benzoyl-N-(3,4-dichlorphenyl)-amino)-propionsäureethylester
2-(N-Benzoyl-3-chlor-4-fluorphenylamino)-propionsäure-methylester
2-(N-Benzoyl-3-chlor-4-fluorphenylamino)-propionsäureisopropylester
2-Chlor-4-ethylamino-6-isopropylamino-1,3,5-triazin
2-Chlor-4-ethylamino-6-(amino-2'-propionitril)-1,3,5-triazin
2-Chlor-4,6-bisethylamino-1,3,5-triazin
2-Chlor-4,6-bisisopropylamino-1,3,5-triazin
2-Chlor-4-isopropylamino-6-cyclopropylamino-1,3,5-triazin
2-Azido-4-methylamino-6-isopropylamino-1,3,5-triazin
2-Methylthio-4-ethylamino-6-isopropylamino-1,3,5-triazin
2-Methylthio-4-ethylamino-6-tert.butylamino-1,3,5-triazin
2-Methylthio-4,6-bisethylamino-1,3,5-triazin
2-Methylthio-4,6-bisisopropylamino-1,3,5-triazin
2-Methoxy-4-ethylamino-6-isopropylamino-1,3,5-triazin
2-Methoxy-4,6-bisethylamino-1,3,5-triazin
2-Methoxy-4,6-bisisopropylamino-1,3,5-triazin
4-Amino-6-tert.butyl-3-methylthio-4,5-dihydro-1,2,4- -triazin-5-on
4-Amino-6-phenyl-3-methyl-4,5-dihydro-1,2,4-triazin-5-on
4-Isobutylidenamino-6-tert.butyl-3-methylthio-4,5-dihydro-1,2,4-triazin-5-on
1-Methyl-3-cyclohexyl-6-dimethylamino-1,3,5-triazin-2,4-dion
3-tert.Butyl-5-chlor-6-methyluracil
3-Isopropyl-5-brom-6-methyluracil
3-sec.Butyl-5-brom-6-methyluracil

3-Cyclohexyl-5,6-trimethylenuracil
2-Methyl-4-(3'-trifluormethylphenyl)-tetrahydro-1,2,4-oxadiazin-3,5-dion
2-Methyl-4-(4'-fluorphenyl)-tetrahydro-1,2,4-oxadiazin-3,5-dion
3-Amino-1,2,4-triazol
1-(4-Chlorphenoxy-3,3-dimethyl-1-(H-1,2,4-triazolyl)-2-butanon
N,N-Diallylchloracetamid
N-Isopropyl-2-chloracetanilid
N-(1-Methyl-propin-2-yl)-2-chloracetanilid
2-Methyl-6-ethyl-N-ethoxymethyl-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracet-anilid
2-Methyl-6-ethyl-N-(pyrazolyl-methyl)-2-chloracetanilid
2,6-Dimethyl-N-(pyrazolyl-methyl)-2-chloracetanilid
2,6-Dimethyl-N-(4-methylpyrazolyl-methyl)-2-chloracetanilid
2,6-Dimethyl-N-(1,2,4-triazolyl-methyl)-2-chloracetanilid
2,6-Dimethyl-N-(3,5-dimethylpyrazolyl-methyl)-2-chloracetanilid
2,6-Dimethyl-N-(1,3-dioxolan-2-yl-methyl)-2-chloracetanilid
2,6-Dimethyl-N-(2-methoxyethyl)-2-chloracetanilid
2,6-Dimethyl-N-isobutoxymethyl-2-chloracetanilid
2,6-Diethyl-N-methoxymethyl-2-chloracetanilid
2,6-Diethyl-N-(n-butoxymethyl)-2-chloracetanilid
2,6-Diethyl-N-ethoxycarbonylmethyl-2-chloracetanilid
2,3-Dimethyl-N-isopropyl-2-chloracetanilid
2,6-Diethyl-N-(2-n-propoxy-ethyl)-2-chloracetanilid
alpha-(2-Methyl-4-chlorphenoxy)-N-methoxy-acetamid
2-(alpha-Naphthoxy)-N,N-diethylpropionamid
2,2-Diphenyl-N,N-dimethylacetamid
alpha-(3,4,5-Tribrompyrazolyl)-N,N-dimethylpropionamid
N-(1,1-Dimethylprop-2-inyl)-3,5-dichlorbenzamid
N-Naphth-1-yl-phthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
5-Acetamido-2,4-dimethyltrifluormethan-sulfonanilid
5-Acetamido-4-methyl-trifluormethan-sulfonanilid
2-Propionyl-amino-4-methyl-5-chlor-thiazol
O-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Diiod-4-hydroxy-benzonitril (Salze)
3,5-Dibrom-4-hydroxy-O-2,4-dinitrophenylbenzaldoxim (Salze)
Pentachlorphenyl-Natriumsalz
2,4-Dichlorphenyl-4'-nitrophenylether
2,4,6-Trichlorphenyl-4'-nitrophenylether
2-Fluor-4,6-dichlorphenyl-4'-nitrophenylether
2-Chlor-4-trifluormethylphenyl-4'-nitrophenylether
2,4'-Dinitro-4-trifluormethyl-diphenylether
2,4-Dichlorphenyl-3'-methoxy-4'-nitro-phenylether
2-Chlor-4-trifluormethylphenyl-3'-ethoxy-4'-nitro-phenylether
2-Chlor-4-trifluormethylphenyl-3'-carboxy-4'-nitro-phenylether (Salze)
2,4-Dichlorphenyl-3'-methoxycarbonyl-4'-nitro-phenylether
2-(3,4-Dichlorphenyl)-4-methyl-1,2,4-oxadiazolidin-3,5-dion
2-(3-Isopropylcarbamoyl-oxyphenyl)-4-methyl-1,2,4-oxadiazolidin-3,5-dion
2-Phenyl-3,1-benzoxazinon-(4)
3-(4-Bromphenyl)-3,4,5,9,10-pentaazatetracyclo-[5,4,1,$0^{2,6}$,$0^{8,11}$]-dodeca-3,9-dien
2-Ethoxy-2,3-dihydro-3,3-dimethyl-5-benzofuranyl-methan-sulfonat
2-Methyl-4,6-dinitrophenol (Salze, Ester)
3-(4-Chlorphenyl)-3,4,5,9,10-pentaazatetracyclo-[5,4,1,$0^{2,6}$,$0^{8,1}$]-dodeca-3,9-dien
2-sec.Butyl-4,6-dinitrophenol (Salze, Ester)
2-sec.Butyl-4,6-dinitrophenol-acetat
2-tert.Butyl-4,6-dinitrophenol-acetat
2-tert.Butyl-4,6-dinitrophenol (Salze)
2-tert.Butyl-5-methyl-4,6-dinitrophenol (Salze)

2-tert.Butyl-5-methyl-4,6-dinitrophenol-acetat
2-sec.Amyl-4,6-dinitrophenol (Salze, Ester)
1-(alpha,alpha-Dimethylbenzyl)-3-(4-methylphenyl)-harnstoff
1-Phenyl-3-(2-methylcyclohexyl)-harnstoff
1-(4-Chlorphenyl)-3,3-dimethyl-harnstoff
1-(4-Chlorphenyl)-3-methyl-3-(butin-1-yl-3)-harnstoff
1-(3,4-Dichlorphenyl)-3,3-dimethyl-harnstoff
1-(3,4-Dichlorphenyl)-3-methyl-3-n-butyl-harnstoff
1-(4-i-Propylphenyl)-3,3-dimethyl-harnstoff
1-(3-Trifluormethylphenyl)-3,3-dimethyl-harnstoff
1-(alpha,alpha,beta,beta-Tetrafluorethoxyphenyl)-3,3-dimethyl-harnstoff
1-(3-tert.Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-[4(4'-Chlorphenoxy)-phenyl]-3,3-dimethyl-harnstoff
1-[4(4'-Methoxyphenoxy)-phenyl]-3,3-dimethyl-harnstoff
1-Cyclooctyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl-harnstoff
Imidazolidin-2-on-1-carbonsäure-isobutylamid
1,2-Dimethyl-3,5-diphenylpyrazolium-methylsulfat
1,3-Dimethyl-4-(3,4-dichlorbenzoyl)-5-(4-methylphenylsulfonyloxy)-pyrazol
2,3,5-Trichlor-pyridinol-(4)
1-Methyl-3-phenyl-5-(3'-trifluormethylphenyl)-pyridon-(4)
1-Methyl-4-phenyl-pyridiniumchlorid
1,1-Dimethylpyridiniumchlorid
1,1'-Dimethyl-4,4'-dipyridylium-di(methylsulfat)
1,1'-Di(3,5-dimethylmorpholin-carbonylmethyl)-4,4'-dipyridylium-dichlorid
1,1'-Ethylen-2,2'-dipyridylium-dibromid
3-[1-(N-Ethoxyamino)-propyliden]-6-ethyl-3,4-dihydro-2-H-pyran-2,4-dion
3-[1-(N-Allyloxyamino)-propyliden]-6-ethyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamino-butyliden]-5,5-dimethylcyclohexan-1,3-dion (Salze)
2-[1-(N-Allyloxyamino-butyliden]-5,5-dimethyl-4-methoxycarbonyl-cyclohexan-1,3-dion (Salze)
2,4-Dichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)
Alpha-Naphthoxyessigsäuremethylester
2-(2,4-Dichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure
(Salze, Ester)
Gibbelerinsäure (Salze)
Dinatrium-methylarsonat
Mononatriumsalz der Methylarsonsäure
N-Phosphon-methyl-glycin (Salze)
N,N-Bis(phosphonmethyl)-glycin (Salze)
2-Chlorethanphosphonsäure-2-chlorethylester
Ammonium-ethyl-carbamoyl-phosphonat
O,O-Di-n-butyl-(1-n-butylamino-cyclohexyl)-phosphonat
Trithiobutylphosphit
O,O-Diisopropyl-5-(2-benzolsulfonylamino-ethyl)-phosphordithioat
5-tert.Butyl-3-(2,4-dichlor-5-isopropoxyphenyl)-1,3,4-oxadiazolon-(2)
4,5-Dichlor-2-trifluormethyl-benzimidazol (Salze)

1,2,3,6-Tetrahydropyridazin-3,6-dion (Salze)
Bernsteinsäure-mono-N,N-dimethylhydrazid (Salze)
(2-Chlorethyl)-trimethyl-ammoniumchlorid
(2-Methyl-4-phenylsulfonyl)-trifluormethansulfonanilid
Ammoniumrhodanid
Calciumcyanamid
2-Chlor-4-trifluormethylphenyl-3'-ethoxycarbonyl-4'-nitrophenylether
1-(4-Benzyloxyphenyl)-3-methyl-3-methoxyharnstoff
2-[1-(2,5-Dimethylphenyl)-ethylsulfonyl]-pyridin-N-oxid
N-Benzyl-N-isopropyl-trimethylacetamid
2-[4-(4'-Chlorphenoxymethyl)-phenoxy]-propionsäuremethylester
2-[4-(5'-Brompyridyl-2-oxy)-phenoxy]-propionsäureethylester
2-[4-(5'-Iodpyridyl-2-oxy)-phenoxy]-propionsäure-n-butylester
2-Chlor-4-trifluormethylphenyl-3'-(2-fluorethoxy)-4'-nitro-phenylether
2-Chlor-4-trifluormethylphenyl-3'-ethoxycarbonylmethylthio-4'-nitrophenylether
2,4,6-Trichlorphenyl-3'-ethoxycarbonyl)methylthio-4'-nitrophenylether
2-[1-(N-Ethoxyamino)-butyliden]-5-(2-ethylthiopropyl)-3-hydroxy-cyclohexen-(2)-on-(1) (Salze)
2-[1-(N-Ethoxyamino)-butyliden]-5-(2-phenylthiopropyl)-3-hydroxy-cyclohexen-(2)-on-(1) (Salze)
4-[4-(4'-Trifluormethyl)-phenoxy]-penten-2-carbonsäureethylester
2-Chlor-4-trifluormethyl-3'-methoxycarbonyl-4'-nitrophenylether
2,4-Dichlorphenyl-3'-carboxy-4'-nitrophenylether (Salze)
4,5-Dimethoxy-2-(3-alpha,alpha,beta-trifluor-beta-bromethoxyphenyl)-3-(2H)-pyridazinon
2,4-Dichlor-3'[2-(2-ethoxy-ethoxy)-ethoxy]-4'-nitro-diphenyl-ether
2,3-Dihydro-3,3-dimethyl-5-benzofuranyl-ethansulfonat
N-[4-Methoxy-6-methyl-1,3,5-triazin-2-yl-aminocarbonyl]-2-chlorbenzolsulfonamid
1-(3-Chlor-4-ethoxyphenyl)-3,3-dimethylharnstoff
2-Methyl-4-chlorphenoxy-thioessigsäureethylester
2-Chlor-3,5-diiod-4-acetoxy-pyridin
1-(4-[2-(4-Methylphenyl)-ethoxy]-phenyl)-3-methyl-3-methoxyharnstoff
2,6-Dimethyl-N-(pyrazolyl-methylenoxymethyl)-2-chloracetanilid
2-Methyl-6-ethyl-N-(pyrazolyl-methylenoxymethyl)-2-chloracetanilid
alpha-2,4-Dichlorphenoxy-propionsäure)-(3-methoxycarbonylamino)-anilid
1-(alpha-2-Brom-4-chlorphenoxypropionsäure)-3-(O-methylcarbamoyl)-anilid
2-Methyl-6-ethyl-N-(pyrazolyl-ethylenoxymethyl)-2-chloracetanilid
2-(3-Trifluormethylphenyl)-4H-3,1-benzoxazin-4-on
2-(3-Pentafluorethoxyphenyl)-4H-3,1-benzoxazin-4-on
2-(3-Trifluormethylthio-phenyl)-4H-3,1-benzoxazin-4-on
2-(3-Difluorchlormethoxyphenyl)-4H-3,1-benzoxazin-4-on
5-Nitro-2-(3-trifluormethyl-phenyl)-4H-3,1-benzoxazin-4-on
5-Chlor-2-(3-trifluormethoxyphenyl)-4H-3,1-benzoxazin-4-on
5-Chlor-2-(3-alpha,alpha,beta,beta-tetrafluorethoxyphenyl)-4H-3,1-benzoxazin-4-on
5-Fluor-2-(3-alpha,alpha,beta,beta-tetrafluorethoxyphenyl)-4H-3,1-benzoxazin-4-on
5-Chlor-2-(4-difluorchlormethoxyphenyl)-4H-3,1-benzoxazin-4-on
5-Fluor-2-(4-difluorchlormethoxyphenyl)-4H-3,1-benzoxazin-4-on
5-Fluor-2-phenyl-4H-3,1-benzoxazin-4-on
5-Fluor-2-(3-difluormethoxyphenyl)-4H-3,1-benzoxazin-4-on
5-Chlor-2-phenyl-4H-3,1-benzoxazin-4-on
N-3-Chlor-4-isopropylphenyl-thiolcarbaminsäuremethylester
6-Methyl-3-methoxy-5,6,dihydro-1,2,4,6-thiatriazin-5-on-1,1-dioxid Natriumsalz
6-Methyl-3-ethoxy-5,6-dihydro-1,2,4,6-thiatriazin-5-on-1,1-dioxid
5-Amino-4-chlor-2-phenyl-3(2H)-pyridazinon
5-Amino-4-brom-2-phenyl-3(2H)-pyridazinon
5-Methylamino-4-chlor-2-(3-trifluormethylphenyl)-3(2H)-pyridazinon
5-Methylamino-4-chlor-2-(3-alpha,alpha,beta,beta-tetrafluorethoxyphenyl)-3(2H)-pyridazinon
5-Dimethylamino-4-chlor-2-phenyl-3(2H)-pyridazinon
4,5-Dimethoxy-2-phenyl-3(2H)-pyridazinon
1-[3'-(2"-Chlor-4"-trifluormethylphenoxy)]-phenyl-4,5-dimethoxy-pyridazinon-6
1-[4'-(3"-Trifluormethyl-phenoxy)]-phenyl-4,5-dimethoxy-pyridazinon-6
N-[4-(4'-Methoxy-phenoxy)-3-chlor-phenyl]-carbaminsäuremethylester
N-[4-(4'-Difluormethoxy-phenoxy)-3-chlor-phenyl]-thio-carbaminsäuremethylester
N-[4-(4'-Difluormethoxy-phenoxy)-phenyl]thio-carbaminsäuremethylester
1-[4-(4'-Methylphenylpropyl)-phenyl]-3-methyl-3-methoxyharnstoff
1-[3-(4'-Chlorphenyl-propyl)-phenyl]-3-methyl-3-methoxyharnstoff
1-[4-(3-Phenyl-2-methyl-propyl)-phenyl]-3-methyl-3-methoxyharnstoff
1-[4-(3-(4'-Chlorphenyl)-2-methyl-propyl)-phenyl]-3-methyl-3-methoxyharnstoff

1-[4-(3-(4'-Methylphenyl)-2-methylpropyl)-phenyl]-3-methyl-3-methoxyharnstoff
2-[1-(N-Ethyloxyamino)-butyliden]-5(4-ethylphenyl)-3-hydroxy-cyclohexen-(2)-on-(1) (Salze)
2-[1-(N-Ethyloxyamino)-butyliden]-5(4-fluorphenyl)-3-hydroxy-cyclohexen-(2)-on-(1) (Salze)
2-[1-(N-Ethyloxyamino)-butyliden]-5-(4-chlorphenyl)-3-hydroxy-cyclohexen-(2)-on-(1) (Salze)
2'-(2,4,6-Trichlorphenyl)-hydrazino-2-cyanacrylsäuremethylester
2-[1-(N-Ethyloxamino)-butyliden]-5-(1,3,3-trimethyl-cyclohexen-1-yl-2)-3-hydroxy-cyclohexen-(2)-on-(1) (Salze)
2-[1-(N-Ethyloxamino)-butyliden]-5-(2,4,4-trimethyl-cyclohexen-1-yl-3)-3-hydroxy-cyclohexen-(2)-on-(1) (Salze)
2-[1-(N-3-Chlorallyl-oxamino)-butyliden]-5-(1-methyl-cyclohex-1-en-4-yl)-3-hydroxy-cyclohexen-(2)-on-(1) (Salze)
3-Isobutoxy-5-methyl-4-methoxycarbonyl-pyrazol
5-Amino-1-(2,4,6-trichlorphenyl)-4-cyano-pyrazol
5-Amino-1-(2,4,6-tribromphenyl)-4-cyano-pyrazol
5-Amino-1-(2,4,6-trichlorphenyl)-4-methoxycarbonyl-pyrazol
5-Amino-1-(2,4-dichlor-6-bromphenyl)-4-methoxycarbonyl-pyrazol
5-Amino-1-(2,6-dichlor-4-bromphenyl)-4-methoxycarbonyl-pyrazol
5-Chlor-2-(3-trifluormethyl-phenyl)-4H-3,1-benzoxazin-4-on
5-Fluor-2-(3-trifluormethyl-phenyl)-4H-3,1-benzoxazin-4-on
2-(3-Tetrafluorethoxyphenyl)-4H-3,1-benzoxazin-4-on
5-Chlor-2-(4'-fluorphenyl)-4H-3,1-benzoxazin-4-on
5-Fluor-2-(4'-fluorphenyl)-4H-3,1,benzoxazin-4-on
5-Fluor-2-(3'fluorphenyl)-4H-3,1-benzoxazin-4-on
5-Chlor-2-(3'-fluorphenyl)-4H-3,1-benzoxazin-4-on
5-Chlor-2-(3'-difluorchlormethylphenyl)-4H-3,1-benzoxazin-4-on
5-Fluor-2-(3'-difluorchlormethylphenyl)-4H-3,1-benzoxazin-4-on
6-Methyl-3-methoxy-5-(4'-nitrophenoxy)-6H-1,2,4,6-thiatriazin-1,1-dioxid
6-Methyl-3-methoxy-5-(propargyloxy-6H-1,2,4,6-thiatriazin-1,1-dioxid
6-Methyl-3-methoxy-5-(2,4-dichlorbenzoxy)-6H-1,2,4,6-thiatriazin-1,1-dioxid
2-(2',4'-Dichlorphenoxy)-2-fluorpropionsäure (Salze, Ester)
2-[4-(5'-Trifluormethylpyridyl-2-oxy)-phenoxy]-propionsäurebutylester
2-[4-(3'-Chlor-5'-trifluormethylpyridyl-2-oxy)-phenoxy]-propionsäure (Salze, Ester)
2-[4-(6-Chlorchinoxalyl-2-oxy)-phenoxy]-propionsäure-pentylester
2-[4-(6-Chlor-chinoxalyl-2-oxy)-phenoxy]-propionsäuremethylester
2-[4-(6-Chlorbenzthiazolyl-2-oxy)-phenoxy]-propionsäure (Salze, Ester)
2-[4-(6-Chlorbenzoxazolyl-2-oxy)-phenoxy]-propionsäure (Salze, Ester)
1-[5-(3-Fluorbenzylthio)-thiadiazolyl-2]-1-methylharnstoff
2-Methoxycarbonyl-N-(3,5-dimethylpyrimidinyl-2-aminocarbonyl)-benzolsulfonamid
alpha-(3,5,6-Trichlor-pyrid-2-yl-oxy)-essigsäure (Salze, Ester)
alpha-(4-Amino-3,5-dichlor-6-fluor-pyrid-2-yl-oxy)-essigsäure (Salze, Ester)
S-[N-(4-Chlorphenyl)-N-isopropyl-carbamoyl-methyl]-O,O-dimethyl-dithiophosphat
Ammonium-(3-amino-3-carboxy-propyl)-methylphosphinat
(Hydroxy)-(methyl)-phosphinyl-L-alpha-aminobutyryl-L-alanyl-Natriumsalz
4-Trifluormethyl-diphenylether
2-(3,5-Dichlorphenyl)-2-(2'2'2'-trichlorethyl)-oxiran
2,4-Diamino-5-methylthio-6-chlor-pyrimidin
N-(4-Ethylthio-2-trifluormethyl-phenyl)-methylsulfonamid
3-Methoxy-4-methyl-5(3-methyl-2-butenyloxy)-1,2-di(hydroxymethyl)-benzol
2-(3,5-Dimethylphenoxy)-2-(1,2,4-triazolyl-1)-essigsäure-N-tertiär-butylamid
2-(3,5-Dichlorphenoxy)-2-(1,2,4-triazolyl-1)-essigsäure-N-tertiär-butylamid
3,7-Dichlor-8-chinolincarbonsäure (Salze, Ester)
5-(2-Chlor-4-trifluormethyl-phenoxy)-2-nitro-N-(1-methoxycarbonyl-ethoxy)-benzamid
N-[3-(1-Ethyl-1-methylpropyl)-isoxazolyl-5]-2,6-dimethoxybenzamid
2'-Methoxyethyl-2-[5-(2-chlor-4-trifluormethyl-phenoxy)-2-nitrophenoxy]-propionat
Methyl-6-(4-isopropyl-4-methyl-5-oxo-2-imidazolin-2-yl)-3-methylbenzoat
Methyl-6-(4-isopropyl-4-methyl-5-oxo-2-imidazolin-2-yl)-4-methylbenzoat
Benzyltrimethylammoniumchlorid
1-[alpha-(4-Trifluormethyl-phenoxy)-phenoxy-propionsäure]-3-(O-methylcarbamoyl)-anilid
1-Dodecyl-cycloheptan-2-on
N-[2-Chlor-4-methylsulfonyl-phenyl]-chlormethansulfonamid
N-[2-Brom-4-ethylsulfonyl-phenyl]-chlormethansulfonamid
N-[2,3-Dichlor-4-ethylsulfonyl-phenyl]-chlormethansulfonamid
2-[1-(N-Ethoxyamino)-pyropyliden-]-5-(pyrid-3-yl)-3-hydroxy-cyclohex-2-en-1-on (Salze)
2,4,5-Trichlorphenoxyessigsäure (Salze, Ester, Amide)
2-[1-(N-Ethoxyamino)-butyliden]-5-(tetrahydropyran-3-yl)-3-hydroxy-cyclohex-2-en-1-on (Salze)

2-[1-(N-Ethoxyamino)-butyliden]-5-(4-methyl-tetrahydropyran-3-yl)-3-hydroxy-cyclohex-2-en-1-on (Salze)
2-[1-(N-Ethoxyamino)-butyliden]-5-(tetrahydrothiopyran-3-yl)-3-hydroxy-cyclohex-2-en-1-on (Salze)
2-[1-(N-Ethoxyamino)-propyliden]-5-(pyrid-3-yl)-3 hydroxy-cyclohex-2-en-1-on (Salze)
2-[1-(N-Allyloxamino)-propyliden]-5-(pyrid-3-yl)-3-hydroxy-cyclohex-2-en-1-on
2-[1-(N-Ethoxyamino)-butyliden]-5-(pyrid-3-yl)-3-hydroxy-cyclohex-2-en-1-on (Salze)
2-[1-(N-Allyloxyamino)-butyliden]-5-(pyrid-3-yl)-3-hydroxy-cyclohex-2-en-1-on (Salze)
2-[4,5-Dihydro-4-methyl-4-isopropyl-5-oxo-1H-imidazol-2-yl]-3-chinolincarbonsäure
2-[4,5-Dihydro-4-methyl-4-isopropyl-5-oxo-1H-imidazol-2-yl]-nicotinsäure-isopropylaminsalz
2-Chlor-2'-methyl-6'-ethyl-N-(N'-1-methoxycarbonyl)-ureidomethylacetanilid
2-Chlor-2'-6'-diethyl-N-(N'-1-methoxycarbonyl)-ureidomethylacetanilid
2-Chlor-2'-6'-dimethyl-N-(N'-1-methoxycarbonyl)-ureidomethylacetanilid
2-Chlor-6-nitro-3-phenoxy-anilin
N-Phosphonomethyl-glycin-trimethyl-sulfoniumsalz
5-(2-Chlor-4-trifluormethyl-phenoxy)-2-nitro-N-methansulfonyl-benzamid
5-(2-Chlor-4-trifluormethyl-phenoxy)-2-nitro-benzoesäure-1-ethoxycarbonyl-ethyl)-ester
1-[3'-(2"-Chlor-4"-trifluormethyl-phenyl-thio)-phenyl]-4,5-dimethoxy-pyridazon-(6)
3-Methyl-6-fluor-5H-thiazolo[2,3-b]-chinazolin-5-on
3-Methyl-2-sulfosäure-5H-thiazolo[2,3-b]-chinazolin-5-on
3-Methyl-2-brom-5H-thiazolo[2,3-b]-chinazolin-5-on
5H-Triazolo[2,3-b]-chinazolin-5-on
2-(1-Ethoxyamino-butyliden)-5-cyclododeca-1,5-dion-9-yl-cyclohex-1-en-1-on
2-(1-Ethoxyamino-butyliden)-5-cyclododecyl-cyclohex-1-en-1-on
5-(4'-Trifluormethyl-2'-chlor-phenoxy)-2-nitro-benzylthioessigsäure-(2-trimethylsilylethyl)-ester
2-(2-Chlorbenzyl)-4,4-dimethyl-isoxazolidin-3-on
N-[4-(3,4-Dichlorbenzyloxymethyl)-phenyl]-N'-methyl-N'-methoxy-harnstoff
N-[4-(4-Trifluormethylbenzyloxymethyl)-phenyl]-N'-methyl-N'-methoxy-harnstoff
N-[3-Chlor-4-(1-benzyloxy-ethyl)-phenyl]-N'-methyl-N'-methoxy-harnstoff
N-[4-(4-Trifluormethyl-benzyloxymethyl)-phenyl]-N',N'-dimethyl-harnstoff
3-(2'-Chlor-4'-trifluormethylphenoxy)-6-nitro-benzensäure-N-methyl-sulfenamid
3-(2'-Chlor-4'-trifluormethylphenoxy)-6-nitro-benzensulfensäureisopropylester
1-Methyl-4-isopropyl-2-(2-methylbenzyloxy)-exo-7-oxabicyclo-[2.2.1]heptan
5-(2-Chlor-4-trifluormethylphenoxy)-2-nitro-acetophenonoxim-O-essigsäuremethylester
O-(3-Phenyl-6-chlorpyridazin-4-yl)-S-n-octyl-thiolcarbonat
3-Methyl-7-chlor-chinolin-8-carbonsäure (Salze, Ester)
3-Ethyl-7-chlor-chinolin-8-carbonsäure (Salze, Ester)
2,6-Diethyl-N-(but-2-inyl)-2-chloracetanilid
2-Chlor-4-trifluormethyl-3'-[(3"-carboxy-propionyl)-hydrazino]-4'-nitro-diphenylether (Natriumsalz)
N-[(4-Chlor-6-methoxy-pyrimidin-2-yl)-aminocarbonyl]-2-ethoxycarbonyl-benzolsulfonamid
N-[(4-Methoxy-6-methyl-1,3,5-triazin-2-yl)-aminocarbonyl]-2-methoxycarbonyl-benzolsulfonamid
2-[1-(N-Ethoxyamino)-butyliden]-5-(4a,7,8,8a-tetrahydro-2H,5H-pyrano[4,3-b]-pyran-3-yl)-3-hydroxy-cyclohexen-2-en-1-on
2-[1-(N-Allyloxamino)-butyliden]-5-(4a,7,8,8a-tetrahydro-2H,5H-pyrano[4,3-b]-pyran-3-yl)-3-hydroxy-cyclohexen-2-en-1-on
5-(2-Chlor-4-trifluormethyl-phenoxy)-2-nitro-benzoesäure-1-ethoxycarbonyl-ethyl)-ester
1-[3'-(2"-Chlor-4"-trifluormethyl-phenyl-thio)-phenyl]-4,5-dimethoxy-pyridazon-(6)
3-Methyl-6-fluor-5H-thiazolo[2,3-b]-chinazolin-5-on
3-Methyl-2-sulfosäure-5H-thiazolo[2,3-b]-chinazolin-5-on
3-Methyl-2-brom-5H-thiazolo[2,3-b]-chinazolin-5-on
5H-Triazolo[2,3-b]-chinazolin-5-on
2-(1-Ethoxyamino-butyliden)-5-cyclododeca-1,5-dion-9-yl-cyclohex-1-en-1-on
2-(1-Ethoxyamino-butyliden)-5-cyclododecyl-cyclohex-1-en-1-on
5-(4'-Trifluormethyl-2'-chlor-phenoxy)-2-nitro-benzylthioessigsäure-(2-trimethylsilylethyl)-ester
2-(2-Chlorbenzyl)-4,4-dimethyl-isoxazolidin-3-on
N-[4-(3,4-Dichlorbenzyloxymethyl)-phenyl]-N'-methyl-N'-methoxy-harnstoff
N-[4-(4-Trifluormethylbenzyloxymethyl)-phenyl]-N'-methyl-N'-methoxy-harnstoff
N-[3-Chlor-4-(1-benzyloxy-ethyl)-phenyl]-N'-methyl-N'-methoxy-harnstoff
N-[4-(4-Trifluormethyl-benzyloxymethyl)-phenyl]-N',N'-dimethyl-harnstoff
3-(2'-Chlor-4'-trifluormethylphenoxy)-6-nitro-benzensäure-N-methyl-sulfenamid
3-(2'-Chlor-4'-trifluormethylphenoxy)-6-nitro-benzensulfensäureisopropylester
1-Methyl-4-isopropyl-2-(2-methylbenzyloxy)-exo-7-oxabicyclo-[2.2.1]heptan
5-(2-Chlor-4-trifluormethylphenoxy)-2-nitro-acetophenonoxim-O-essigsäure-methylester
O-(3-Phenyl-6-chlorpyridazin-4-yl)-S-n-octyl-thiolcarbonat
3-Methyl-7-chlor-chinolin-8-carbonsäure (Salze, Ester)
3-Ethyl-7-Chlor-chinolin-8-carbonsäure (Salze, Ester)

2,6-Diethyl-N-(but-2-inyl)-2-chloracetanilid
2-Chlor-4-trifluormethyl-3'-[(3"-carboxy-propionyl)-hydrazino]-4'-nitro-diphenylether (Natriumsalz)
N-[(4-Chlor-6-methoxy-pyrimidin-2-yl)-aminocarbonyl]-2-ethoxycarbonyl-benzolsulfonamid
N-[(4-Methoxy-6-methyl-1,3,5-triazin-2-yl)-aminocarbonyl]-2-methoxycarbonyl-benzolsulfonamid
2-[1-(N-Ethoxyamino)-butyliden]-5-(4a,7,8,8a-tetrahydro-2H,5H-pyrano[4,3-b]-pyran-3-yl)-3-hydroxy-cyclohexen-2-en-1-on
2-[1-(N-Allyloxamino)-butyliden]-5-(4a,7,8,8a-tetrahydro-2H,5H-pyrano[4,3-b]-pyran-3-yl)-3-hydroxy-cyclohexen-2-en-1-on
2-[1-(N-Allyloxamino)-butyliden]-5-(3,4,4a,7,8,8a-hexahydro-2H,5H-pyrano[4,3-b]-pyran-3-yl)-3-hydroxy-cyclohexen-2-en-1-on
2-[1-(N-Ethoxyamino)-butyliden]-5-(3,4,4a,7,8,8a-hexahydro-2H,5H-pyrano[4,3-b]-pyran-3-yl)-3-hydroxy-cyclohexen-2-en-1-on
2-[4,5-Dihydro-4-methyl-4-isopropyl-5-oxo-1H-imidazol-2-yl]-5-ethyl-pyridin-3-carbonsäure
2-(1-Ethoxyamino-butyliden)-5-(5,6-dihydro-2H-1,1-dioxothiopyran-3-yl)-3-hydroxy-cyclohex-2-1-on
2-(1-Ethoxyamino-propyliden)-5-(5,6-dihydro-2H-1,1-dioxothiopyran-3-yl)-3-hydroxy-cyclohex-2-en-1-on
2-(1-Propargylamino-butyliden)-5-(5,6-dihydro-2H-1,1-dioxothiopyran-3-yl)-3-hydroxy-cyclohex-2-en-1-on)
3-(2'-Chlor-4'-trifluormethylphenoxy)-6-nitro-phenylglyoxylsäuremethylester
2-[4-(2-Chlor-4-trifluormethylphenoxy)-phenoxy]-propionsäurebutylester
2-Carboxy-N-[[(4-methoxy-6-chlorpyrimidin-2-yl)-amino]-carbonyl]-benzol-sulfonamid

Außerdem kann es von Nutzen sein, die Isoharnstoffe der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

## Patentansprüche

1. Isoharnstoffe der allgemeinen Formel I,

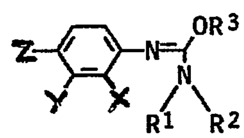

I

in der die Substituenten folgende Bedeutung haben:
$R^1$ eine $C_1$–$C_3$-Alkylgruppe,
$R^2$ eine $C_1$–$C_3$-Alkylgruppe oder eine Methoxygruppe,
$R^3$ ein Phenylrest, der ein oder zwei Halogenatome und/oder $C_1$–$C_5$-Alkylgruppen tragen kann, wobei der Phenylrest durch ein oder zwei der folgenden Gruppen substituiert ist: $C_1$–$C_4$-Alkoxy, $C_1$–$C_3$-Halogenalkoxy, 1-($C_2$–$C_3$-Alkoxycarbonyl)-$C_1$–$C_2$-alkoxy und/oder $-NHR^4$ oder
ein Benzylrest, der bis zu drei der folgenden Gruppen tragen kann: Halogen, Hydroxy, Nitro, $C_1$–$C_5$-Alkyl, $C_1$–$C_4$-Alkoxy, $C_1$–$C_3$-Halogenalkoxy, 1-($C_2$–$C_3$-Alkoxycarbonyl)-$C_1$–$C_2$-alkoxy und/oder $-NHR^4$, wobei
$R^4$ eine $C_2$–$C_4$-Alkoxycarbonylgruppe, eine $C_2$–$C_4$-Alkylcarbonylgruppe, eine $C_4$–$C_7$-Cycloalkylcarbonylgruppe, eine N,N-Di-$C_1$–$C_4$-alkylaminocarbonylgruppe oder eine $C_2$–$C_3$-Alkoxycarbonyl-$C_1$–$C_4$-alkylgruppe, wobei diese Gruppen bis zu drei Halogenatome tragen können, bedeutet
und
X, Y und Z unabhängig voneinander: Wasserstoff, Halogenatom, $C_1$–$C_4$-Alkylgruppe, $C_1$–$C_4$-Alkoxygruppe, $C_1$–$C_3$-Halogenalkylgruppe, Phenoxygruppe und/oder Phenyl-$C_1$–$C_4$-alkoxygruppe, wobei die aromatischen Gruppen bis zu drei der folgenden Substituenten tragen können: Halogen und/oder $C_1$–$C_4$-Alkyl.

2. Isoharnstoffe der Formel I gemäß Anspruch 1, dadurch gekennzeichnet, daß X Wasserstoff bedeutet.

3. Isoharnstoffe der Formel I gemäß Anspruch 1, dadurch gekennzeichnet, daß $R^2$ Methoxy bedeutet.

4. Verfahren zur Herstellung von Isoharnstoffen der Formel I gemäß Anspruch 1, dadurch gekennzeichnet, daß man einen N-Arylchlorformimidsäurearylester der Formel

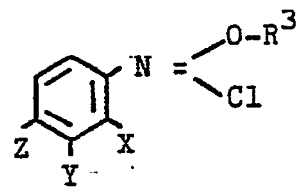

(II),

in der X, Y, Z und $R^3$ die im Anspruch 1 genannten Bedeutungen haben, mit einem Amin der Formel

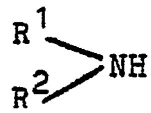

(III),

in der $R^1$ und $R^2$ die im Anspruch 1 genannten Bedeutungen haben, umsetzt.

5. Verfahren zur Herstellung von Isoharnstoffen der Formel I gemäß Anspruch 1, dadurch gekennzeichnet, daß man ein Chlorformamidin der Formel

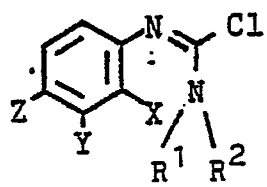

(IV),

in der X, Y, Z, $R^1$ und $R^2$ die im Anspruch 1 genannten Bedeutungen haben, mit einem Phenol der Formel

$R^3$–OH (V),

in der $R^3$ die im Anspruch 1 genannten Bedeutungen hat, in Gegenwart eines säurebindenden Mittels umsetzt.

6. Herbizid, enthaltend einen Isoharnstoff der Formel I gemäß Anspruch 1.

7. Herbizid, enthaltend einen Isoharnstoff der Formel I gemäß Anspruch 2.

8. Herbizid, enthaltend inerte Zusatzstoffe und 0,1 bis 95 Gew.-% eines Isoharnstoffs der Formel I gemäß Anspruch 1.

9. Verfahren zur Bekämpfung unerwünschten Pflanzenwuchses, dadurch gekennzeichnet, daß man die unerwünschten Pflanzen oder von unerwünschtem Pflanzenwuchs freizuhaltende Fläche mit einer herbizid wirksamen Menge eines Isoharnstoffs der Formel I gemäß Anspruch 1 behandelt.

**Claims**

1. An isourea of the general formula I

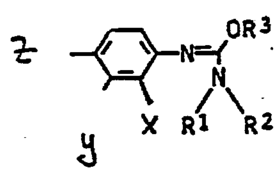

(I)

where $R^1$ is $C_1$–$C_3$-alkyl, $R^2$ is $C_1$–$C_3$-alkyl or methoxy, $R^3$ is phenyl which can carry one or two halogen atoms and/or $C_1$–$C_5$-alkyl, in which the phenyl radical is substituted by one or two of the following groups: $C_1$–$C_4$-alkoxy, $C_1$–$C_3$-haloalkoxy, 1-($C_2$- or $C_3$-alkoxycarbonyl)-$C_1$–$C_2$-alkoxy and/or –$NHR^4$ or a benzyl radical which can carry up to three of the following groups: halogen, hydroxyl, nitro, $C_1$–$C_5$-alkyl, $C_1$–$C_4$-alkoxy, $C_1$–$C_3$-haloalkoxy, 1-($C_2$- or $C_3$-alkoxycarbonyl)-$C_1$–$C_2$-alkoxy and/or –$NHR^4$, where $R^4$ is $C_2$–$C_4$-alkoxycarbonyl, $C_2$–$C_4$-alkylcarbonyl, $C_4$–$C_7$-cycloalkylcarbonyl, N,N-di-$C_1$–$C_4$-alkylaminocarbonyl or $C_2$- or $C_3$-alkoxycarbonyl-$C_1$–$C_4$-alkyl, it being possible for these groups to carry up to three halogen atoms, and X, Y and Z independently of one another are each hydrogen, halogen, $C_1$–$C_4$-alkyl, $C_1$–$C_4$-alkoxy, $C_1$–$C_3$-haloalkyl, phenoxy and/or phenyl-$C_1$–$C_4$-alkoxy, it being possible for the aromatic groups to carry up to three of the following substituents: halogen and/or $C_1$–$C_4$-alkyl.

2. An isourea of the formula I as claimed in claim 1, where X is hydrogen.

3. An isourea of the formula I as claimed in claim 1, where $R^2$ is methoxy.

4. A process for the preparation of an isourea of the formula I as claimed in claim 1, wherein an aryl N-arylchloroformimidate of the formula

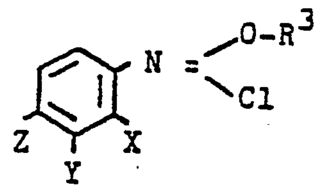

(II)

where X, Y, Z and $R^3$ have the meanings given in claim 1, is reacted with an amine of the formula

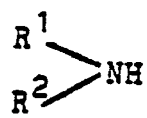

(III)

where $R^1$ and $R^2$ have the meanings given in claim 1.

5. A process for the preparation of an isourea of the formula I as claimed in claim 1, wherein a chloroformamidine of the formula

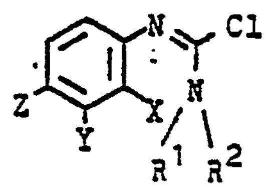

(IV)

where X, Y, Z, $R^1$ and $R^2$ have the meanings given in claim 1, is reacted with a phenol of the formula

$R^3$–OH (V)

where $R^3$ has the meanings given in claim 1, in the presence of an acid acceptor.

6. A herbicide containing an isourea of the formula I as claimed in claim 1.

7. A herbicide containing an isourea of the formula I as claimed in claim 2.

8. A herbicide containing inert additives and from 0.1 to 95% by weight of an isourea of the formula I as claimed in claim 1.

9. A process for controlling undesirable plant growth, wherein the undesirable plants or the area to be kept free from undesirable plant growth are or is treated with a herbicidally effective amount of an isourea of the formula I as claimed in claim 1.

## Revendications

1. Isourées de formule générale I

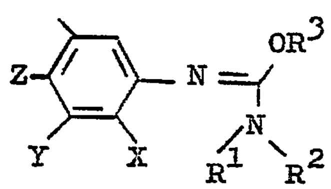

I

dans laquelle les substituants ont la signification suivante

$R^1$, un groupe alkyle en $C_1$–$C_3$,

$R^2$, un groupe alkyle en $C_1$–$C_3$ ou un groupe méthoxy,

$R^3$, un reste phényle, qui peut porter un ou deux atomes d'halogène et/ou des groupes alkyle en $C_1$–$C_5$, le reste phényle étant substitué par un ou deux des groupes suivants:
alcoxy en $C_1$–$C_4$, halogénalcoxy en $C_1$–$C_3$, 1-(alcoxycarbonyl en $C_2$–$C_3$)-alcoxy en $C_1$–$C_2$ et/ou –$NHR^4$
ou
un reste benzyle, qui peut porter jusqu'à trois des groupes suivants: halogène, hydroxy, nitro, alkyle

en $C_1$–$C_5$, alcoxy en $C_1$–$C_4$, halogénoalcoxy en $C_1$–$C_3$, 1-(alcoxycarbonyl en $C_2$–$C_3$)-alcoxy en $C_1$–$C_2$ et/ou $-NHR^4$ ou
$R^4$ représentant un groupe alcoxycarbonyle en $C_2$–$C_4$, un groupe alkylcarbonyle en $C_2$–$C_4$, un groupe cycloalkylcarbonyle en $C_4$–$C_7$, un groupe N,N-di-alkylaminocarbonyle en $C_1$–$C_4$ ou un groupe alcoxycarbonyle en $C_2$–$C_3$-alkyle en $C_1$–$C_4$, ces groupes pouvant porter jusqu'à trois atomes d'halogène, et
X, Y et Z indépendant les uns des autres: hydrogène, atome d'halogène, groupe alkyle en $C_1$–$C_4$, groupe alcoxy en $C_1$–$C_4$, groupe halogéno-alkyle en $C_1$–$C_3$, groupe phénoxy et/ou groupe phényl-alcoxy en $C_1$–$C_4$, les groupes aromatiques pouvant porter jusqu'à trois des substituants suivants: halogène, et/ou alkyle en $C_1$–$C_4$.

2. Isourées de formule I selon la revendication 1, caractérisées par le fait que X représente hydrogène.

3. Isourées de formule I selon la revendication 1, caractérisées par le fait que $R^2$ représente méthoxy.

4. Procédé de préparation d'isourées de formule I selon la revendication 1, caractérisé par le fait que l'on fait réagir un ester arylique de N-arylchloroformimidoacide de formule

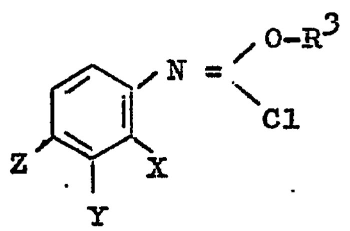 (II),

dans laquelle X, Y, Z et $R^3$ ont les significations susindiquées avec une amine de formule

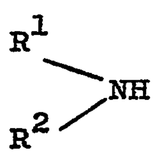 (III),

dans laquelle $R^1$ et $R^2$ ont les significations données dans la revendication 1.

5. Procédé de préparation d'isourées de formule I selon la revendication 1, caractérisé par le fait que l'on fait réagir une chloroformamidine de formule

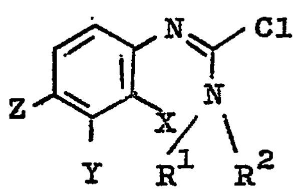 (IV),

dans laquelle X, Y, Z, $R^1$ et $R^2$ ont les significations données dans la revendication 1, avec un phénol de formule

$$R^3\text{–}OH \quad (V),$$

dans laquelle $R^3$ a les significations données dans la revendication 1, en présence d'un liant acide.

6. Herbicide contenant une isourée de formule I selon la revendication 1.

7. Herbicide contenant une isourée de formule I selon la revendication 2.

8. Herbicide contenant des additifs inertes et 0,1 à 95% en poids d'une isourée de formule I selon la revendication 1.

9. Procédé de lutte contre la croissante des plantes indésirables, caractérisé par le fait que l'on traite les plantes indésirables ou les surfaces à maintenir exemptes de la croissance de plantes indésirables avec une quantité efficace du point de vue herbicide d'une isourée de formule I selon la revendication 1.